(19) Europäisches Patentamt
European Patent Office
Office européen des brevets

(11) EP 3 145 865 B1

(12) # EUROPEAN PATENT SPECIFICATION

(45) Date of publication and mention of the grant of the patent:
**02.12.2020 Bulletin 2020/49**

(21) Application number: **15726462.3**

(22) Date of filing: **15.05.2015**

(51) Int Cl.:
*C01B 39/02* (2006.01) *C01B 39/46* (2006.01)
*B01D 53/22* (2006.01) *C07C 1/20* (2006.01)
*C10G 11/05* (2006.01) *B01D 53/02* (2006.01)
*B01D 53/94* (2006.01) *B01J 29/74* (2006.01)
*B01J 29/76* (2006.01) *B01J 35/10* (2006.01)
*C07C 209/16* (2006.01) *C07C 5/27* (2006.01)
*C10G 29/20* (2006.01) *C10G 45/62* (2006.01)
*C10G 45/64* (2006.01) *C10G 47/14* (2006.01)
*C10G 50/00* (2006.01)

(86) International application number:
**PCT/US2015/031010**

(87) International publication number:
**WO 2015/179228 (26.11.2015 Gazette 2015/47)**

(54) **PROCESSES USING MOLECULAR SIEVE SSZ-95**

VERFAHREN UNTER VERWENDUNG DES MOLEKULARSIEBS SSZ-95

PROCÉDÉS UTILISANT UN TAMIS MOLÉCULAIRE SSZ-95

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.05.2014 US 201462001108 P**

(43) Date of publication of application:
**29.03.2017 Bulletin 2017/13**

(73) Proprietor: **Chevron U.S.A. Inc.**
**San Ramon, California 94583 (US)**

(72) Inventors:
- **OJO, Adeola Florence**
  **San Ramon, California 94583 (US)**
- **ZHANG, Yihua**
  **San Ramon, California 94583 (US)**
- **ZONES, Stacey Ian**
  **San Ramon, California 94583 (US)**
- **LEI, Guan-Dao**
  **San Ramon, California 94583 (US)**

(74) Representative: **Haseltine Lake Kempner LLP**
**Redcliff Quay**
**120 Redcliff Street**
**Bristol BS1 6HU (GB)**

(56) References cited:
**EP-A1- 2 438 985**
**EP-A2- 1 063 014**
**WO-A1-2013/092707**
**WO-A2-2005/042144**
**WO-A2-2007/146622**
**US-A- 5 053 373**
**US-A- 5 252 527**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present disclosure relates to new crystalline molecular sieve SSZ-95, a method for preparing SSZ-95, and uses for SSZ-95.

### BACKGROUND

Because of their unique sieving characteristics, as well as their catalytic properties, crystalline molecular sieves and zeolites are especially useful in applications such as hydrocarbon conversion, gas drying and separation. Although many different crystalline molecular sieves have been disclosed, there is a continuing need for new molecular sieves with desirable properties for gas separation and drying, hydrocarbon and chemical conversions, and other applications. New molecular sieves may contain novel internal pore architectures and acid site properties, providing enhanced selectivities and activities in these processes.

Molecular sieves are classified by the Structure Commission of the International Zeolite Association according to the rules of the IUPAC Commission on Zeolite Nomenclature. According to this classification, framework type zeolites and other crystalline microporous molecular sieves, for which a structure has been established, are assigned a three letter code and are described in the "Atlas of Zeolite Framework Types" Sixth Revised Edition, Elsevier (2007).

Molecular sieves are periodically ordered in three dimensions. Structurally disordered structures show periodic ordering in dimensions less than three (i.e., in two, one or zero dimensions). This phenomenon is characterized as stacking disorder of structurally invariant Periodic Building Units (PerBuU). Crystal structures built from Periodic Building Units are called end-member structures if periodic ordering is achieved in all three dimensions. Disordered structures are those where the stacking sequence of the Periodic Building Units deviates from periodic ordering up to statistic stacking sequences.

Molecular sieves having a MTT-type framework code have a one-dimensional 10-ring pore system. MTT-type molecular sieves have very similar, but not identical, X-ray diffraction patterns. SSZ-32 and its small crystal variant, SSZ-32x, are known MTT-type molecular sieves.

SSZ-32x, in comparison with standard SSZ-32, has broadened X-ray diffraction peaks that may be a result of its inherent small crystals, altered Argon adsorption ratios, increased external surface area and reduced cracking activity over other intermediate pore size molecular sieves used for a variety of catalytic processes. SSZ-32x and methods for making it are disclosed in U.S. Pat. Nos. 7,390,763, 7,569,507 and 8,545,805.

Known methods for making SSZ-32 and SSZ-32x employ high temperature calcination steps before the ion-exchange step for the purpose of removing extra framework cations. For Example, in Example 2 of U.S. Patent No. 8,545,805, the as-made SSZ-32x product was calcined at 595°C prior to undergoing ammonium ion-exchange. Likewise, in Example 2 of U.S. Patent No. 7,390,763, the as-made SSZ-32x produce was calcined at 1100°F (593°C) prior to undergoing ammonium ion-exchange.

WO 2005/042144 describes a method of making SSZ-32X.

WO 2007/146622 describes molecular sieve SSZ-75 having STI topology prepared using a tetramethylene-1,4-bis-(N-methylpyrrolidinium) dication as a structure-directing agent.

However, it has now been found that by using the manufacturing method described herein below, a novel molecular sieve designated herein as SSZ-95 is achieved. SSZ-95 is characterized as having a unique acid site density which causes the molecular sieve to exhibit enhanced selectivity, and less gas-make (e.g. production of $C_1$-$C_4$ gases), compared to conventional SSZ-32x materials.

### SUMMARY

The present disclosure is directed to a family of crystalline molecular sieves with unique properties and a MTT-type topology, referred to herein as "molecular sieve SSZ-95" or simply "SSZ-95."

The present invention provides: a process for converting hydrocarbons as set out in claim 1; a process for the production of light olefins as set out in claim 10, a process for producing methylamine or dimethylamine as set out in claim 11; a process for separating gases as set out in claim 12; a process for treating a cold-start engine exhaust gas stream as set out in claim 13; and a process for the reduction of oxides of nitrogen as set out in claim14.

Described herein is molecular sieve SSZ-95 characterized as having:

(a) a mole ratio of 20 to 70 of silicon oxide to aluminum oxide,
(b) a total micropore volume of between 0.005 and 0.02 cc/g; and
(c) a H-D exchangeable acid site density of up to 50% relative to SSZ-32.

Also described herein is a process for preparing SSZ-95 by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

## DETAILED DESCRIPTION

Introduction

The following terms will be used throughout the specification and will have the following meanings unless otherwise indicated.
The term "active source" means a reagent or precursor material capable of supplying at least one element in a form that can react and which can be incorporated into the molecular sieve structure. The terms "source" and "active source" can be used interchangeably herein.
The term "molecular sieve" and "zeolite" are synonymous and include (a) intermediate and (b) final or target molecular sieves and zeolites produced by (1) direct synthesis or (2) post-crystallization treatment (secondary modification). Secondary synthesis techniques allow for the synthesis of a target material from an intermediate material by heteroatom lattice substitution or other techniques. For example, an aluminosilicate can be synthesized from an intermediate borosilicate by post-crystallization heteroatom lattice substitution of the Al for B. Such techniques are known, for example as described in U.S. Patent No. 6,790,433 to C.Y. Chen and Stacey Zones, issued September 14, 2004.
The term "MTT molecular sieve" includes all molecular sieves and their isotypes that have been assigned the International Zeolite Associate framework code MTT, as described in the Atlas of Zeolite Framework Types, eds. Ch. Baerlocher, L.B. McCusker and D.H. Olson, Elsevier, 6th revised edition, 2007.
The term "SSZ-32x" refers to a molecular sieve characterized as having (a) a silica-to-alumina ratio of 20 to 70, (b) small, broad lathe-like crystallites in the range of less than 1,000 Angstroms, typically 200-400 Angstroms, and (c) an Argon adsorption ratio of between 0.55 and 0.70. The Argon adsorption ratio (ArAR) is calculated as follows:

$$ArAR = \frac{\text{Ar adsorption at 87K between the relative pressures of 0.001 and 0.1}}{\text{total Ar adsorption up to relative pressure of 0.1}}$$

The term "relative to SSZ-32" means as compared to SSZ-32 material made per the teachings of Example 1 of U.S. Patent No. 5,252,527 to Zones, calcined per the teachings of Example 8 of that patent.
The term "pre-calcination" and its past tense form "pre-calcined" refer to the step of calcining a molecular sieve prior to the sieve undergoing an ion-exchange step to remove extra framework cations.
The term "post-calcination" and its past tense form "post-calcined" refer to the step of calcining a molecular sieve after to the sieve has undergone an ion-exchange step to remove extra framework cations.
It will be understood by a person skilled in the art that the MTT-type molecular sieve materials made according to the process described herein may contain impurities, such as amorphous materials.
The term "full decomposition temperature" refers to the minimum temperature, as identified by thermogravimetric analysis, indicating the onset and the end of organic template decomposition.
The term "Periodic Table" refers to the version of IUPAC Periodic Table of the Elements dated Jun. 22, 2007, and the numbering scheme for the Periodic Table Groups is as described in Chem. Eng. News, 63(5), 26-27 (1985).
For the purposes of this specification and appended claims, unless otherwise indicated, all numbers expressing quantities, percentages or proportions, and other numerical values used in the specification and claims, are to be understood as being modified in all instances by the term "about." Accordingly, unless indicated to the contrary, the numerical parameters set forth in the following specification and attached claims are approximations that can vary depending upon the desired properties sought to be obtained. It is noted that, as used in this specification and the appended claims, the singular forms "a," "an," and "the," include plural references unless expressly and unequivocally

limited to one referent. As used herein, the term "include" and its grammatical variants are intended to be non-limiting, such that recitation of items in a list is not to the exclusion of other like items that can be substituted or added to the listed items. As used herein, the term "comprising" means including elements or steps that are identified following that term, but any such elements or steps are not exhaustive, and an embodiment can include other elements or steps.

Unless otherwise specified, the recitation of a genus of elements, materials or other components, from which an individual component or mixture of components can be selected, is intended to include all possible sub-generic combinations of the listed components and mixtures thereof.

The patentable scope is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

All numerical ranges stated herein are inclusive of the lower and upper values stated for the range, unless stated otherwise.

Properties for materials described herein, where reported, are determined as follows:

(a) $SiO_2/Al_2O_3$ Ratio (SAR): determined by ICP elemental analysis. A SAR of infinity (∞) represents the case where there is no aluminum in the zeolite, i.e., the mole ratio of silica to alumina is infinity. In that case the molecular sieve is comprised of essentially all of silica.
(b) Surface area: determined by $N_2$ adsorption at its boiling temperature. BET surface area is calculated by the 5-point method between $P/P_0$ of 0.05 and 0.2. Samples are first pre-treated at 400°C for up to 24 hours in the presence of flowing, dry $N_2$ so as to eliminate any adsorbed volatiles like water or organics.
(c) Micropore volume: determined by $N_2$ adsorption at its boiling temperature. Micropore volume is calculated by the t-plot method between $P/P_0$ of 0.015 and 0.40. Samples are first pre-treated at 400°C for up to 24 hours in the presence of flowing dry $N_2$ so as to eliminate any adsorbed volatiles like water or organics.
(d) Pour point: temperature at which an oil will begin to flow under controlled conditions, as determined by ASTM D5950-12a.
(e) API gravity: the gravity of a petroleum feedstock/product relative to water, as determined by ASTM D4052-11.
(f) Viscosity index (VI): an empirical, unit-less number indicated the effect of temperature change on the kinematic viscosity of the oil. The higher the VI of a base oil, the lower its tendency to change viscosity with temperature. Determined by ASTM 2270-04.
(g) Acid site distribution: Acid sites distribution determined by H-D exchange FTIR adapted from the published description by E.J.M Hensen, D. G. Poduval, D.A.J Michel Ligthart, J.A. Rob van Veen, M. S. Rigutto, J.Phys. Chem. C.114, 8363-8374 2010.

Preparation of SSZ-95

As will be described herein below, SSZ-95 is prepared by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02.

In general, SSZ-32x is synthesized from a reaction mixture suitable for synthesizing the MTT-type molecular sieve. Methods for synthesizing MTT-type molecular sieves, including SSZ-32 and SSZ-32x, are described in U.S. Patent Nos. 5,053,373; 5,252,527; 5,397,454; 5,707,601; 5,785,947; 6,099,820; 7,157,075; 7,390,763; 7,468,126; 7,569,507; 7,682,600; 7,824,658; and 8,545,805.

The as-made, structure directing agent-containing aluminosilicate SSZ-32x is subjected to a "pre-calcination" step, as defined herein above. The pre-calcination step can be performed at atmospheric pressure or under vacuum, in the presence of oxygen (air) or in an inert atmosphere. The temperature(s) selected for the pre-calcination step should be less than the full decomposition temperature of the organic structure directing agent (e.g. as determined by TGA),

and well below a temperature which, for the pre-calcination temperature and time period selected, would result in the complete removal of all of the organic material.

Following the pre-calcination step, the molecular sieve is characterized as follows: (a) a micropore volume of between 0.002 and 0.015 cc/g; (b) an external surface area of between 215 and 250 $m^2/g$; and (c) a BET surface area of between 240 and 280 $m^2/g$. In one subembodiment, the micropore volume is between 0.005 and 0.014 cc/g. In another subembodiment, the micropore volume is between 0.006 and 0.013 cc/g.

Following the pre-calcination step, the molecular sieve will undergo an ion-exchange step to remove the Group 1 and/or 2 extra-framework cations (e.g. $K^+$) and replace them with hydrogen, ammonium, or any desired metal-ion.

Following the ion-exchange step, the ion-exchanged molecular sieve is subjected to a "post-calcination" step (as defined herein above) at one or more temperatures between 95 and 500°C for between 1 and 16 hours. In one embodiment, the crystals are post-calcined at one or more temperatures between 120 and 490°C for between 1 and 6 hours. The post-calcination step can be performed at atmospheric pressure or under vacuum, in the presence of oxygen (air) or in an inert atmosphere.

The cumulative weight loss (CWL) during the pre- and post-calcination steps should be greater than zero and less than or equal to 10 wt.% ($0 < CWL \leq 10$ wt.%). In one subembodiment, the CWL will be between 4 and 9 wt.%. In another subembodiment, the CWL is between 5 and 8.5 wt.%.

Following the post-calcination step, the molecular sieve is characterized as follows: (a) a total micropore volume of between 0.005 and 0.02 cc/g; (b) an external surface area of between 200 and 250 $m^2/g$; and (c) a BET surface area of between 240 and 280 $m^2/g$. In one subembodiment, the total micropore volume is between 0.008 and 0.018 cc/g. In another subembodiment, the total micropore volume is between 0.008 and 0.015 cc/g.

The temperature profile (e.g. the heating and cooling rates) for the pre- and post-calcination steps will depend somewhat on the calcination equipment employed. For example, in a commercial zeolite production facility, belt calciners with the capability of subjecting the zeolite to multiple temperatures are often employed to calcine zeolites. Commercial belt calciners may employ multiple and independent calcination zones, allowing the sieve product to be subjected to multiple temperatures as the material travels through the oven. One skilled in the art can readily select the belt speed and oven temperature(s) in order to calcine the SSZ-95 product to yield crystals that contain the target decomposition residue concentration with desired micropore volume.

The method of the present invention leaves a decomposition residue on the SSZ-95 zeolite crystals following the post-calcination step. While not wishing to be bound by any particular theory, it is believed that the decomposition residue selectively impacts the ion-exchangeable sites thereby resulting in a more preferred acid site density and acid site location, to yield a finished hydroisomerization catalyst having a unique acid site density that exhibits a greater degree of isomerization selectivity and less gas make (i.e. production of $C_1$-$C_4$ gases) than conventional catalysts containing known MTT-type materials.

The molecular sieve made from the process disclosed herein can be formed into a wide variety of physical shapes. Generally speaking, the molecular sieve can be in the form of a powder, a granule, or a molded product, such as extrudate having a particle size sufficient to pass through a 2-mesh (Tyler) screen and be retained on a 400-mesh (Tyler) screen. In cases where the catalyst is molded, such as by extrusion with an organic binder, the molecular sieve can be extruded before drying, or, dried or partially dried and then extruded.

The molecular sieve can be composited with other materials resistant to the temperatures and other conditions employed in organic conversion processes. Such matrix materials include active and inactive materials and synthetic or naturally occurring zeolites as well as inorganic materials such as alumina, clays, silica and metal oxides. Examples of such materials and the manner in which they can be used are disclosed in U.S. Patent Nos. 4,910,006 and 5,316,753.

The extrudate or particle may then be further loaded using a technique such as impregnation, with one or more active metals selected from the group consisting of metals from Groups 8 to 10 of the Periodic Table, to enhance the hydrogenation function. It may be desirable to co-impregnate a modifying metal and one or more Group 8 to 10 metals at once, as disclosed in U.S. Pat. No. 4,094,821. In one embodiment, the at least one active metal is selected from the group consisting of nickel, platinum, palladium, and combinations thereof. After metal loading, the metal loaded extrudate can be calcined in air or inert gas at temperatures from 200°C to 500°C. In one embodiment, the metal loaded extrudate is calcined in air or inert gas at temperatures from 390°C to 482°C.

SSZ-95 is useful as catalysts for a variety of hydrocarbon conversion reactions such as hydrocracking, dewaxing, olefin isomerization, alkylation of aromatic compounds and the like. SSZ-95 is also useful as an adsorbent for separations.

Characterization of Molecular Sieve SSZ-95 Molecular sieve SSZ-95 made by the process disclosed herein is characterized as having:

(a) a mole ratio of 20 to 70 of silicon oxide to aluminum oxide,
(b) a total micropore volume of between 0.005 and 0.02 cc/g; and
(c) a H-D exchangeable acid site density of up to 50% relative to SSZ-32.

In one embodiment, SSZ-95 has a H-D exchangeable acid site density of 0.5 to 30% relative to SSZ-32. In another embodiment, SSZ-95 has a H-D exchangeable acid site density of between 2 and 25% relative to SSZ-32.

## Processes Using SSZ-95

### Hydrocarbon Conversion Processes

SSZ-95 is useful as catalyst in a wide range of hydrocarbon conversion processes. Hydrocarbon conversion reactions are chemical and catalytic processes in which carbon-containing compounds are changed to different carbon-containing compounds. Specific examples of hydrocarbon conversion processes which are effectively catalyzed by SSZ-95, by itself or in combination with one or more other catalytically active substances including other crystalline catalysts, include hydrocracking, dewaxing, catalytic cracking, aromatics formation, isomerization, alkylation and transalkylation, conversion of paraffins to aromatics, isomerization of olefins, xylene isomerization, oligomerization, condensation of alcohols, methane upgrading and polymerization of 1-olefins.

The SSZ-95 catalysts can have high selectivity, and under hydrocarbon conversion conditions can provide a high percentage of desired products relative to total products.

For high catalytic activity, SSZ-95 should be predominantly in its hydrogen ion form. Generally, the molecular sieve is converted to its hydrogen form by ammonium exchange followed by calcination. If the molecular sieve is synthesized with a high enough ratio of SDA cation to sodium ion, calcination alone can be sufficient. Typically, after calcination at least 80% of the cation sites are occupied by hydrogen ions and/or rare earth ions. As used herein, "predominantly in the hydrogen form" means that, in its calcined form, at least 80% of the cation sites of the molecular sieve are occupied by hydrogen ions and/or rare earth ions.

SSZ-95 can be used in processing hydrocarbon feedstocks. Hydrocarbon feedstocks contain carbon compounds and can be from many different sources, such as virgin petroleum fractions, recycle petroleum fractions, shale oil, liquefied coal, tar sands oil, synthetic paraffins from NAO, recycled plastic feedstocks. Other feeds include synthetic feeds, such as those derived from a Fischer-Tropsch process, including an oxygenate-containing Fischer-Tropsch process boiling below about 371°C. In general, the feed can be any carbon-containing feedstock susceptible to zeolitic catalytic reactions. Depending on the type of processing the hydrocarbon feed is to undergo, the feed can contain metal or be free of metals; it can also have high or low nitrogen or sulfur impurities. It can be appreciated, however, that in general processing will be more efficient (and the catalyst more active) the lower the metal, nitrogen, and sulfur content of the feedstock.

The conversion of hydrocarbon feeds can take place in any convenient mode, for example, in fluidized bed, moving bed, or fixed bed reactors depending on the types of process desired. The formulation of the catalyst particles will vary depending on the conversion process and method of operation.

The following Table A indicates typical reaction conditions which can be employed when using catalysts comprising SSZ-95 in the hydrocarbon conversion reactions disclosed herein. Typical conditions are indicated in parentheses.

TABLE A

| Process | Temperature (°C) | Pressure (MPa) | LHSV ($h^{-1}$) |
|---|---|---|---|
| Hydrocracking | 175 to 485 | 0.05 to 35 | 0.1 to 30 |
| Catalytic Cracking | 127 to 885 | (0.1 to 0.5) | 0.5 to 50 |
| Dewaxing | 200 to 475<br>(250 to 450) | 0.10 to 20.7[1]<br>(1.38 to 20.7)[1] | 0.1 to 20<br>(0.2 to 10) |
| Aromatics Formation | 400 to 600<br>(480 to 550) | 0.1 to 1 | 0.1 to 15 |
| Paraffins to Aromatics | 100 to 700 | 0 to 6.89[1] | 0.5 to 40[5] |
| Isomerization | 93 to 538<br>(204 to 315) | 0.34 to 6.89[1] | 1 to 10<br>(1 to 4) |
| Xylene Isomerization | 260 to 593[2]<br>(315 to 566)[2]<br>38 to 371[4] | 0.05 to 5.1[2]<br>(0.1 to 0.51)[2]<br>0.1 to 20.3[4] | 0.1 to 100[5]<br>(0.5 to 50)[5]<br>0.5 to 50 |

(continued)

| Process | Temperature (°C) | Pressure (MPa) | LHSV ($h^{-1}$) |
|---|---|---|---|
| Oligomerization | 232 to 649[2]<br>10 to 232[4]<br>(27 to 204)[4] | 0.01 to 5.1[2,3] | 0.2 to 50[2]<br>0.05 to 20[5]<br>(0.1 to 10)[5] |
| Condensation of Alcohols | 260 to 538 | 0.0034 to 6.89[1] | 0.5 to 50[5] |
| [1]Gauge pressure, i.e., the absolute pressure minus the ambient pressure<br>[2]Gas-phase reaction<br>[3]Hydrocarbon partial pressure<br>[4]Liquid-phase reaction<br>[5]Weight Hourly Space Velocity (WHSV) | | | |

Other reaction conditions and parameters are provided below.

Hydrocracking

Using a catalyst which comprises SSZ-95 (e.g., predominantly in the hydrogen form) and a hydrogenation promoter, heavy petroleum residual feedstocks, cyclic stocks and other hydrocrackate charge stocks can be hydrocracked using the process conditions and catalyst components disclosed in U.S. Patent Nos. 4,910,006 and 5,316,753.

The hydrocracking catalysts contain an effective amount of at least one hydrogenation component of the type commonly employed in hydrocracking catalysts. The hydrogenation component is generally selected from the group of hydrogenation catalysts consisting of one or more metals of Groups 6 and 8-10 of the Periodic Table, including the salts, complexes and solutions containing such. The hydrogenation catalyst can be selected from the group of metals, salts and complexes thereof of the group consisting of at least one of platinum, palladium, rhodium, iridium, ruthenium and mixtures thereof or the group consisting of at least one of nickel, molybdenum, cobalt, tungsten, titanium, chromium and mixtures thereof. Reference to the catalytically active metal or metals is intended to encompass such metal or metals in the elemental state or in some form such as an oxide, sulfide, halide, carboxylate and the like. The hydrogenation catalyst is present in an effective amount to provide the hydrogenation function of the hydrocracking catalyst, for example in the range of from 0.05 to 25 wt. %.

Catalytic Cracking

Hydrocarbon cracking stocks can be catalytically cracked in the absence of hydrogen using SSZ-95, for example predominantly in the hydrogen form.

When SSZ-95 is used as a catalytic cracking catalyst in the absence of hydrogen, the catalyst can be employed in conjunction with traditional cracking catalysts, e.g., any aluminosilicate heretofore employed as a component in cracking catalysts. Typically, these are large pore, crystalline aluminosilicates. Examples of these traditional cracking catalysts are disclosed in the aforementioned U.S. Patent Nos. 4,910,006 and 5,316,753. When a traditional cracking catalyst component is employed, the relative weight ratio of the traditional cracking catalyst component to the SSZ-95 is generally from 1:10 to 500:1, e.g., from 1:10 to 200:1, from 1:2 to 50:1, or from 1:1 to 20:1. SSZ-95 and/or the traditional cracking component can be further ion exchanged with rare earth ions to modify selectivity.

The cracking catalysts are typically employed with an inorganic oxide matrix component. See the aforementioned U.S. Patent Nos. 4,910,006 and 5,316,753 for examples of such matrix components.

Hydrotreating

SSZ-95 is useful as a hydrotreating catalyst. During hydrotreatment, oxygen, sulfur and nitrogen present in a hydrocarbon feed is reduced to low levels. Aromatics and olefins, if present in the feed, can also have their double bonds saturated. In some cases, the hydrotreating catalyst and hydrotreating conditions are selected to minimize cracking reactions, which can reduce the yield of the most desulfided product (typically useful as a fuel).

Hydrotreating conditions typically include a reaction temperature of from 204°C to 482°C, e.g., from 343°C to 454°C; a pressure of from 500 to 5000 psig (3.5 to 34.5 MPa), e.g., from 1000 to 3000 psig (6.89 to 20.7 MPa); a liquid hourly space velocity (LHSV) of from 0.5 to 20 $h^{-1}$ (v/v); and an overall hydrogen consumption of from 300 to 2000 SCF/bbl (standard cubic feet per barrel) of liquid hydrocarbon feed (53.4 to 356 $m^3$ $H_2/m^3$ feed). The hydrotreating catalyst will typically be a composite of a Group 6 metal or compound thereof and a Group 8-10 metal or compound

thereof supported on the molecular sieve disclosed herein. Typically, such hydrotreating catalysts are presulfided.

Catalysts useful for hydrotreating hydrocarbon feeds are disclosed in U. S. Patent Nos. 4,347,121 and 4,810,357. Suitable catalysts include noble metals from Group 8-10 of the Periodic Table, such as iron, cobalt, nickel, platinum or palladium, and/or Group 6 metals, such as chromium, molybdenum, or tungsten. Examples of combinations of Group 8-10 and Group 6 metals include Ni-Mo or Ni-W. Other suitable catalysts are described in U. S. Patent Nos. 3,904,513 and 4,157,294. U.S. Patent No. 3,852,207 describes suitable noble metal catalysts and mild hydrotreating conditions.

The amount of hydrogenation component(s) in the catalyst suitably range from 0.5 to 10 wt. % of Group 8-10 component(s) and from 5 to 25 wt. % of Group 6 metal component(s), calculated as metal oxide(s) per 100 parts by weight of total catalyst, where the percentages by weight are based on the weight of the catalyst before sulfiding. The hydrogenation component(s) in the catalyst can be in the oxide and/or sulfide form.

Dewaxing

SSZ-95, for example predominantly in the hydrogen form, can be used to dewax hydrocarbon feeds by selectively removing straight chain paraffins. Typically, the viscosity index of the dewaxed product is improved (compared to the waxy feed) when the waxy feed is contacted with SSZ-95 under isomerization dewaxing conditions.

The catalytic dewaxing conditions are dependent in large measure on the feed used and upon the desired pour point. Hydrogen is typically present in the reaction zone during the catalytic dewaxing process. The hydrogen to feed ratio is typically from 500 to 30,000 SCF/bbl (0.089 to 5.34 SCM/L (standard cubic meters/liter)), e.g., from 1000 to 20,000 SCF/bbl (0.178 to 3.56 SCM/L). Generally, hydrogen will be separated from the product and recycled to the reaction zone. Typical feedstocks include light gas oil, heavy gas oils and reduced crudes boiling above about 177°C.

A typical dewaxing process is the catalytic dewaxing of a hydrocarbon oil feedstock boiling above about 177°C and containing straight chain and slightly branched chain hydrocarbons by contacting the hydrocarbon oil feedstock in the presence of added hydrogen gas at a hydrogen pressure of from 15 to 3000 psi (103 kPa to 20.7 MPa) with a catalyst comprising SSZ-95 and at least one Group 8-10 metal. The term "Group 8-10 metal" as used herein is meant the metal itself or a compound thereof.

The SSZ-95 dewaxing catalyst can optionally contain a hydrogenation component of the type commonly employed in dewaxing catalysts. See the aforementioned U.S. Patent Nos. 4,910,006 and 5,316,753 for examples of these hydrogenation components.

The hydrogenation component is present in an effective amount to provide an effective hydrodewaxing and hydroisomerization catalyst, for example in the range of from 0.05 to 5 wt. %. The catalyst can be run in such a mode to increase isomerization dewaxing at the expense of cracking reactions.

The feed can be hydrocracked, followed by dewaxing. This type of two stage process and typical hydrocracking conditions are described in U.S. Patent No. 4,921,594.

SSZ-95 can also be utilized as a dewaxing catalyst in the form of a combination of catalysts. The combination comprises a first catalyst comprising SSZ-95 and, desirably, at least one metal selected from Groups 8-10 of the Periodic Table, and a second catalyst comprising an aluminosilicate zeolite which is more shape selective than SSZ-95. As used herein, the term "combination" includes mixtures of SSZ-95 and the aluminosilicate zeolite, layers of SSZ-95 and the zeolite, or any other configuration in which the feed comes in contact with both SSZ-95 and the zeolite. The use of combined catalysts in the form of layers is disclosed in U.S. Patent No. 5,149,421. The layering can also include a bed of SSZ-95 layered with a non-zeolitic component designed for either hydrocracking or hydrofinishing.

SSZ-95 can also be used to dewax raffinates, including bright stock, under conditions such as those disclosed in U.S. Patent No. 4,181,598.

It is often desirable to use mild hydrogenation (sometimes referred to as hydrofinishing) to produce more stable dewaxed products. The hydrofinishing step can be performed either before or after the dewaxing step, typically after. Hydrofinishing is typically conducted at a temperature ranging from 190°C to 340°C, at a pressure ranging from 400 to 3000 psig (2.76 to 20.7 MPa), at a LHSV ranging from 0.1 and 20 $h^{-1}$ and a hydrogen recycle rate ranging from 400 to 1500 SCF/bbl (0.071 to 0.27 SCM/L). The hydrogenation catalyst employed must be active enough not only to hydrogenate the olefins, di-olefins and color bodies which can be present, but also to reduce the aromatic content. Suitable hydrogenation catalysts are disclosed in U. S. Patent No. 4,921,594. The hydrofinishing step is beneficial in preparing an acceptably stable product (e.g., a lubricating oil) since dewaxed products prepared from hydrocracked stocks tend to be unstable to air and light and tend to form sludges spontaneously and quickly.

Lube oil can be prepared using SSZ-95. For example, a $C_{20+}$ lube oil can be made by isomerizing a $C_{20+}$ olefin feed over a catalyst comprising SSZ-95 in the hydrogen form and at least one metal selected from Groups 8-10 of the Periodic Table. Alternatively, the lubricating oil can be made by hydrocracking in a hydrocracking zone a hydrocarbon feedstock to obtain an effluent comprising a hydrocracked oil, and catalytically dewaxing the effluent at a temperature of at least 204°C and at a pressure of from 15 to 3000 psig (103 kPa to 20.7 MPa) in the presence of added hydrogen gas with a catalyst comprising SSZ-95 in the hydrogen form and at least one metal selected from Groups 8-10 of the

Periodic Table.

Hydrogenation

SSZ-95 can be used in a catalyst to catalyze hydrogenation of a hydrocarbon feed containing unsaturated hydrocarbons. The unsaturated hydrocarbons can comprise olefins, dienes, polyenes, aromatic compounds and the like.

Hydrogenation is accomplished by contacting the hydrocarbon feed containing unsaturated hydrocarbons with hydrogen in the presence of a catalyst comprising SSZ-95. The catalyst can also contain one or more metals of Group 6 and Group 8-10, including salts, complexes and solutions thereof. Reference to these catalytically active metals is intended to encompass such metals or metals in the elemental state or in some form such as an oxide, sulfide, halide, carboxylate and the like. Examples of such metals include metals, salts or complexes wherein the metal is selected from the group consisting of platinum, palladium, rhodium, iridium or combinations thereof, or the group consisting of nickel, molybdenum, cobalt, tungsten, titanium, chromium, vanadium, rhenium, manganese and combinations thereof.

The hydrogenation component of the catalyst (i.e., the aforementioned metal) is present in an amount effective to provide the hydrogenation function of the catalyst, e.g., in the range of from 0.05 to 25 wt. %.

Hydrogenation conditions, such as temperature, pressure, space velocities, contact time and the like are well known in the art.

Methane Upgrading

Higher molecular weight hydrocarbons can be formed from lower molecular weight hydrocarbons by contacting the lower molecular weight hydrocarbon with a catalyst comprising SSZ-95 and a metal or metal compound capable of converting the lower molecular weight hydrocarbon to a higher molecular weight hydrocarbon. Examples of such reactions include the conversion of methane to $C_{2+}$ hydrocarbons such as ethylene or benzene or both. Examples of useful metals and metal compounds include lanthanide and or actinide metals or metal compounds.

These reactions, the metals or metal compounds employed and the conditions under which they can be run are disclosed in U.S. Patents No. 4,734,537; 4,939,311; 4,962,261; 5,095,161; 5,105,044; 5,105,046; 5,238,898; 5,321,185; and 5,336,825.

Aromatics Formation

SSZ-95 can be used to convert light straight run naphthas and similar mixtures to highly aromatic mixtures. Thus, normal and slightly branched chained hydrocarbons having a boiling range above 40°C and less than 200°C can be converted to products having substantially higher octane aromatics content by contacting the hydrocarbon feed with a catalyst comprising SSZ-95. It is also possible to convert heavier feeds into BTX or naphthalene derivatives of value using a catalyst comprising SSZ-95.

The conversion catalyst typically contains a Group 8-10 metal compound to have sufficient activity for commercial use. The Group 8-10 noble metals and their compounds, platinum, palladium, and iridium, or combinations thereof can be used. Rhenium or tin or a mixture thereof can also be used in conjunction with the Group 8-10 metal compound (typically a noble metal compound), for example a platinum compound. The amount of Group 8-10 metal present in the conversion catalyst should be within the normal range of use in reforming catalysts, e.g., from 0.05 to 2.0 wt. %, or from 0.2 to 0.8 wt. %.

For the selective production of aromatics in useful quantities, it is desirable that the conversion catalyst be substantially free of acidity, for example, by neutralizing the molecular sieve with a basic metal, e.g., an alkali metal compound. Methods for rendering the catalyst free of acidity are known in the art. See the aforementioned U.S. Patent Nos. 4,910,006 and 5,316,753 for a description of such methods.

Typical alkali metals are sodium, potassium, rubidium and cesium. The molecular sieve itself can be substantially free of acidity only at very high silica to alumina mole ratios.

Conversion of Paraffins to Aromatics

SSZ-95 can be used to convert light gas $C_2$ to $C_6$ paraffins to higher molecular weight hydrocarbons including aromatic compounds. Typically, the molecular sieve will contain a catalyst metal or metal oxide wherein the metal is selected from the group consisting of Groups 3, 8-10, 11, and 12 of the Periodic Table, e.g., gallium, niobium, indium or zinc, in the range of from 0.05 to 5 wt. %.

Alkylation and Transalkylation

SSZ-95 can be used in a process for the alkylation or transalkylation of an aromatic hydrocarbon. The process comprises contacting the aromatic hydrocarbon with a $C_2$ to $C_{16}$ olefin alkylating agent or a polyalkyl aromatic hydrocarbon transalkylating agent, under at least partial liquid phase conditions, and in the presence of a catalyst comprising SSZ-95.
SSZ-95 can also be used for removing benzene from gasoline by alkylating the benzene as described above and removing the alkylated product from the gasoline.
For high catalytic activity, SSZ-95 should be predominantly in its hydrogen ion form. It is typical that, in its calcined form, at least 80% of the cation sites of the molecular sieve are occupied by hydrogen ions and/or rare earth ions.
Examples of suitable aromatic hydrocarbon feedstocks which can be alkylated or transalkylated by the process disclosed herein include aromatic compounds such as benzene, toluene and xylene. Benzene is especially useful. There can be occasions where naphthalene or naphthalene derivatives such as dimethylnaphthalene can be desirable. Mixtures of aromatic hydrocarbons can also be employed.
Suitable olefins for the alkylation of the aromatic hydrocarbon are those containing from 2 to 20 carbon atoms, e.g., from 2 to 4 carbon atoms, such as ethylene, propylene, 1-butene, *cis*-2-butene and *trans*-2-butene, or mixtures thereof. There can be instances where pentenes are desirable. Typical olefins are ethylene and propylene. Longer chain alpha-olefins can be used as well.
When transalkylation is desired, the transalkylating agent is a polyalkyl aromatic hydrocarbon containing two or more alkyl groups that each can have from 2 to about 4 carbon atoms. For example, suitable polyalkyl aromatic hydrocarbons include di-, tri- and tetra-alkyl aromatic hydrocarbons, such as diethylbenzene, triethylbenzene, diethylmethylbenzene (diethyltoluene), diisopropylbenzene, diisopropyltoluene, dibutylbenzene, and the like. Typical polyalkyl aromatic hydrocarbons are the dialkylbenzenes. A particularly desirable polyalkyl aromatic hydrocarbon is diisopropylbenzene.
When alkylation is the process conducted, reaction conditions are as follows. The aromatic hydrocarbon feed should be present in stoichiometric excess. It is typical that the molar ratio of aromatics to olefins be greater than four-to-one to prevent rapid catalyst fouling. The reaction temperature can range from 38°C to 315°C, e.g., from 121°C to 232°C. The reaction pressure should be sufficient to maintain at least a partial liquid phase in order to retard catalyst fouling. This is typically 50 to 1000 psig (0.345 to 6.89 MPa) depending on the feedstock and reaction temperature. Contact time can range from 10 seconds to 10 hours, but is usually from 5 minutes to an hour. The WHSV, in terms of grams (pounds) of aromatic hydrocarbon and olefin per gram (pound) of catalyst per hour, is generally within the range of 0.5 to 50 $h^{-1}$.
When transalkylation is the process conducted, the molar ratio of aromatic hydrocarbon will generally range from 1:1 to 25:1, e.g., from 2:1 to 20:1. The reaction temperature can range from 38°C to 315°C, e.g., from 121°C to 232°C. The reaction pressure should be sufficient to maintain at least a partial liquid phase, typically in the range of from 50 to 1000 psig (345 kPa to 6.89 MPa), e.g., from 300 to 600 psig (2.07 to 4.14 MPa). The WHSV will range from 0.1 to 10 $h^{-1}$. U.S. Patent No. 5,082,990 describes such processes.

Xylene Isomerization

SSZ-95 can also be useful in a process for isomerizing one or more xylene isomers in a $C_8$ aromatic feed to obtain *ortho-, meta-,* and para-xylene in a ratio approaching the equilibrium value. In particular, xylene isomerization is used in conjunction with a separate process to manufacture para-xylene. For example, a portion of the para-xylene in a mixed $C_8$ aromatics stream can be recovered by crystallization and centrifugation. The mother liquor from the crystallizer is then reacted under xylene isomerization conditions to restore *ortho-, meta-* and *para*-xylenes to a near equilibrium ratio. At the same time, part of the ethylbenzene in the mother liquor is converted to xylenes or to products which are easily separated by filtration. The isomerate is blended with fresh feed and the combined stream is distilled to remove heavy and light byproducts. The resultant $C_8$ aromatics stream is then sent to the crystallizer to repeat the cycle.
Optionally, isomerization in the vapor phase is conducted in the presence of 3.0 to 30.0 moles of hydrogen per mole of alkylbenzene (e.g., ethylbenzene). If hydrogen is used, the catalyst should comprise from 0.1 to 2.0 wt. % of a hydrogenation/dehydrogenation component selected from a Group 8-10 metal component, especially platinum or nickel.
Optionally, the isomerization feed can contain from 10 to 90 wt. % of a diluent such as toluene, trimethylbenzene, naphthenes or paraffins.

Isomerization of $C_4$ to $C_7$ Hydrocarbons

The present catalyst is highly active and highly selective for isomerizing $C_4$ to $C_7$ hydrocarbons. The activity means that the catalyst can operate at relatively low temperature which thermodynamically favors highly branched paraffins. Consequently, the catalyst can produce a high octane product. The high selectivity means that a relatively

high liquid yield can be achieved when the catalyst is run at a high octane.

The present process comprises contacting the isomerization catalyst, i.e., a catalyst comprising SSZ-95 in the hydrogen form, with a hydrocarbon feed under isomerization conditions. The feed is typically a light straight run fraction, boiling within the range of from -1°C to 121°C, e.g., from 16°C to 93°C. Typically, the hydrocarbon feed for the process comprises a substantial amount of $C_4$ to $C_7$ normal and slightly branched low octane hydrocarbons, for example $C_5$ and $C_6$ hydrocarbons.

The isomerization reaction is typically carried out in the presence of hydrogen. Hydrogen can be added to give a hydrogen to hydrocarbon ratio ($H_2$/HC) of from 0.5 to 10 $H_2$/HC, e.g., from 1 and 8 $H_2$/HC. See the aforementioned U.S. Patent Nos. 4,910,006 and 5,316,753 for a further discussion of isomerization process conditions.

A low sulfur feed is especially useful in the present process. The feed desirably contains less than 10 ppm sulfur, e.g., less than 1 ppm sulfur or less than 0.1 ppm sulfur. In the case of a feed which is not already low in sulfur, acceptable levels can be reached by hydrogenating the feed in a pre-saturation zone with a hydrogenating catalyst which is resistant to sulfur poisoning. See the aforementioned U.S. Patent Nos. 4,910,006 and 5,316,753 for a further discussion of this hydrodesulfurization process.

It is typical to limit the nitrogen level and the water content of the feed. Catalysts and processes which are suitable for these purposes are known to those skilled in the art.

After a period of operation, the catalyst can become deactivated by sulfur or coke. See the aforementioned U.S. Patent Nos. 4,910,006 and 5,316,753 for a further discussion of methods of removing this sulfur and coke and of regenerating the catalyst.

The conversion catalyst desirably contains a Group 8-10 metal compound to have sufficient activity for commercial use. The Group 8-10 noble metals and their compounds, platinum, palladium, and iridium, or combinations thereof can be used. Rhenium and tin can also be used in conjunction with the noble metal. Typically, the metal is platinum. The amount of Group 8-10 metal present in the conversion catalyst should be within the normal range of use in isomerizing catalysts, e.g., from 0.05 to 2.0 wt. %, or from 0.2 to 0.8 wt. %.

## Isomerization of Olefins

SSZ-95 can be used to isomerize olefins. The feed stream is a hydrocarbon stream containing at least one $C_4$ to $C_6$ olefin, e.g., a $C_4$ to $C_6$ normal olefin such as normal butene. Normal butene as used in this specification means all forms of normal butene, e.g., 1-butene, *cis*-2-butene and *trans*-2-butene. Typically, hydrocarbons other than normal butene or other $C_4$ to $C_6$ normal olefins will be present in the feed stream. These other hydrocarbons can include alkanes, other olefins, aromatics, hydrogen, and inert gases.

The feed stream typically can be the effluent from a fluid catalytic cracking unit or a methyl-*tert*-butyl ether (MTBE) unit. A fluid catalytic cracking unit effluent typically contains from 40 to 60 wt. % normal butenes. A MTBE unit effluent typically contains from 40 to 100 wt. % normal butene. The feed stream typically contains at least about 40 wt. % normal butene, e.g., at least 65 wt. % normal butene.

The process is carried out under isomerization conditions. The hydrocarbon feed is contacted in a vapor phase with a catalyst comprising the SSZ-95. The process can be carried out generally at a temperature from 329°C to 510°C for butenes, e.g., from 371°C to 482°C, or from 177°C to 343°C for pentenes and hexenes. The pressure ranges from sub-atmospheric to 200 psig (1379 kPa), e.g., from 15 to 200 psig (103 to 1379 kPa) or from 1 to 150 psig (7 to 1034 kPa).

The LHSV during contacting is generally from 0.1 to 50 $h^{-1}$, based on the hydrocarbon feed, e.g., from 0.1 to 20 $h^{-1}$, from 0.2 to 10 $h^{-1}$, or from 1 to 5 $h^{-1}$. A hydrogen/hydrocarbon molar ratio is maintained from about 0 to 30 or higher. The hydrogen can be added directly to the feed stream or directly to the isomerization zone. The reaction is typically substantially free of water, typically less than 2 wt. % based on the feed. The process can be carried out in a packed bed reactor, a fixed bed, fluidized bed reactor, or a moving bed reactor. The bed of the catalyst can move upward or downward. The mole % conversion of, for example, normal butene to iso-butene is at least 10, e.g., at least 25 or at least 35.

## Oligomerization of Olefins

It is expected that SSZ-95 can also be used to oligomerize straight and branched chain olefins having from 2 to 21 carbon atoms, e.g., from 2 to 5 carbon atoms. The oligomers which are the products of the process are medium to heavy olefins which are useful for both fuels, i.e., gasoline or a gasoline blending stock and chemicals.

The oligomerization process comprises contacting the olefin feedstock in the gaseous or liquid phase with a catalyst comprising SSZ-95. The molecular sieve can have the original cations associated therewith replaced by a wide variety of other cations according to techniques well known in the art. Typical cations would include hydrogen, ammonium and metal cations including mixtures of the same. Of the replacing metallic cations, cations of metals such as rare earth metals, manganese, calcium, as well as metals of Group 12 of the Periodic Table, e.g., zinc, and Group 8-10 of the

Periodic Table, e.g., nickel are particularly desirable. One of the prime requisites is that the molecular sieve has a fairly low aromatization activity, i.e., in which the amount of aromatics produced is not more than about 20 wt. %. This is accomplished by using a molecular sieve with controlled acid activity (alpha value) of from 0.1 to 120, e.g., from 0.1 to 100, as measured by its ability to crack n-hexane.

Alpha values are defined by a standard test known in the art, e.g., as shown in U.S. Patent No. 3,960,978. If required, such molecular sieves can be obtained by steaming, by use in a conversion process or by any other method which can occur to one skilled in the art.

Polymerization of 1-Olefins

SSZ-95 can be used in a catalyst for the polymerization of 1-olefins, e.g., the polymerization of ethylene. To form the olefin polymerization catalyst, SSZ-95 is reacted with a particular type of organometallic compound. Organometallic compounds useful in forming the polymerization catalyst include trivalent and tetravalent organotitanium and organochromium compounds having alkyl moieties and, optionally, halide moieties. In the context of the present disclosure, the term "alkyl" includes both straight and branched chain alkyl, cycloalkyl and alkaryl groups such as benzyl.

Examples of trivalent and tetravalent organochromium and organotitanium compounds are disclosed in U. S. Patent Nos. 4,376,722; 4,377,497; 4,446,243; and 4,526,942.

Examples of the organometallic compounds used to form the polymerization catalyst include, but are not limited to, compounds corresponding to the general formula (1):

$$ZR_cHal_{d-c} \qquad (1)$$

wherein Z is a metal selected from titanium and chromium; R is alkyl; Hal is halogen (e.g., Cl or Br); c is 1-4; and d is greater than or equal to c and is 3 or 4.

Examples of organotitanium and organochromium compounds encompassed by such a formula include compounds of the formula $CrR_4$, $CrR_3$, $CrR_3Hal$, $CrR_2Hal$, $CrR_2Hal_2$, $CrRHal_2$, $CrRHal_3$, $TiR_4$, $TiR_3$, $TiR_3Hal$, $TiR_2Hal$, $TiR_2Hal_2$, $TiRHal_2$, $TiRHal_3$, wherein Hal can be Cl or Br and R can be methyl, ethyl, propyl, isopropyl, butyl, isobutyl, sec-butyl, tert-butyl, pentyl, isopentyl, neopentyl, hexyl, isohexyl, neohexyl, 2-ethybutyl, octyl, 2-ethylhexyl, 2,2-diethylbutyl, 2-isopropyl-3-methylbutyl, etc., cyclohexylalkyls such as, for example, cyclohexylmethyl, 2-cyclohexylethyl, 3-cyclohexylpropyl, 4-cyclohexylbutyl, and the corresponding alkyl-substituted cyclohexyl radicals as, for example, (4-methylcyclohexyl)methyl, neophyl, i.e., β,β-dimethyl-phenethyl, benzyl, ethylbenzyl, and p-isopropylbenzyl.

The organotitanium and organochromium materials employed in the catalyst can be prepared by techniques well known in the art. See, for example the aforementioned U. S. Patent Nos. 4,376,722; 4,377,497; 4,446,243; and 4,526,942.

The organotitanium or organochromium compounds can be with SSZ-95 such as by reacting the organometallic compound and the molecular sieve, in order to form the olefin polymerization catalyst. Generally, such a reaction takes place in the same reaction medium used to prepare the organometallic compound under conditions which promote formation of such a reaction product. The molecular sieve can simply be added to the reaction mixture after formation of the organometallic compound has been completed. Molecular sieve is added in an amount sufficient to provide from 0.1 to 10 parts by weight, e.g., from 0.5 to 5 parts by weight, of organometallic compound in the reaction medium per 100 parts by weight of molecular sieve.

Temperature of the reaction medium during reaction of organometallic compound with molecular sieve is also maintained at a level which is low enough to ensure the stability of the organometallic reactant. Thus, temperatures in the range of from -150°C to 50°C, e.g., from -80°C to 0°C can be usefully employed. Reaction times of from 0.01 to 10 hours, e.g., from 0.1 to 1 hour, can be employed in the reaction of the organotitanium or organochromium compound with the molecular sieve.

Upon completion of the reaction, the catalyst material so formed can be recovered and dried by evaporating the reaction medium solvent under a nitrogen atmosphere. Alternatively, olefin polymerization reactions can be conducted in this same solvent-based reaction medium used to form the catalyst.

The polymerization catalyst can be used to catalyze polymerization of 1-olefins. The polymers produced using the catalysts of this disclosure are normally solid polymers of at least one mono-1-olefin containing from 2 to 8 carbon atoms per molecule. These polymers are normally solid homopolymers of ethylene or copolymers of ethylene with another mono-1-olefin containing 3 to 8 carbon atoms per molecule. Exemplary copolymers include those of ethylene/propylene, ethylene/1-butene, ethylene/1-hexane, and ethylene/1-octene and the like. The major portion of such copolymers is derived from ethylene and generally consists of from 80 to 99 mol % ethylene, e.g., from 95 to 99 mol % ethylene. These polymers are well suited for extrusion, blow molding, injection molding and the like.

The polymerization reaction can be conducted by contacting monomer or monomers, e.g., ethylene alone or with one or more other olefins, and in the substantial absence of catalyst poisons such as moisture and air, with a catalytic

amount of the supported organometallic catalyst at a temperature and at a pressure sufficient to initiate the polymerization reaction. If desired, an inert organic solvent can be used as a diluent and to facilitate materials handling if the polymerization reaction is conducted with the reactants in the liquid phase, e.g. in a particle form (slurry) or solution process. The reaction can also be conducted with reactants in the vapor phase, e.g., in a fluidized bed arrangement in the absence of a solvent but, if desired, in the presence of an inert gas such as nitrogen.

The polymerization reaction is carried out at temperatures of from 30°C or less to 200°C or more, depending to a great extent on the operating pressure, the pressure of the olefin monomers, and the particular catalyst being used and its concentration. Naturally, the selected operating temperature is also dependent upon the desired polymer melt index since temperature is a factor in adjusting the molecular weight of the polymer. Typically, the temperature used is from 30°C to 100°C in a conventional slurry or "particle forming" process or from 100°C to 150°C in a "solution forming" process. A temperature of from 70°C to 110°C can be employed for fluidized bed processes.

The pressure to be used in the polymerization reactions can be any pressure sufficient to initiate the polymerization of the monomer(s) to high molecular weight polymer. The pressure, therefore, can range from sub-atmospheric pressures, using an inert gas as diluent, to super-atmospheric pressures of up to about 30,000 psig (206.8 MPa) or more, for example from atmospheric (0 psig) up to 1000 psig (6.89 MPa). As a general rule, a pressure of 20 to 800 psig (138 kPa to 5.52 MPa) is desirable.

The selection of an inert organic solvent medium to be employed in the solution or slurry process is not too critical, but the solvent should be inert to the supported organometallic catalyst and olefin polymer produced, and should be stable at the reaction temperature used. It is not necessary, however, that the inert organic solvent medium also serves as a solvent for the polymer to be produced. Among the inert organic solvents applicable for such purposes can be mentioned saturated aliphatic hydrocarbons having from 3 to 12 carbon atoms such as hexane, heptane, pentane, isooctane, purified kerosene and the like, saturated cycloaliphatic hydrocarbons having from 5 to 12 carbon atoms such as cyclohexane, cyclopentane, dimethylcyclopentane and methylcyclohexane and the like and aromatic hydrocarbons having from 6 to 12 carbon atoms such as benzene, toluene, xylene, and the like. Particularly desirable solvent media are cyclohexane, pentane, hexane and heptane.

Hydrogen can be introduced into the polymerization reaction zone in order to decrease the molecular weight of the polymers produced (i.e., give a much higher melt index). Partial pressure of hydrogen when hydrogen is used can be within the range of from 5 to 100 psig (34 to 689 kPa), e.g., from 25 to 75 psig (172 to 517 kPa). The melt indices of the polymers produced in accordance with this disclosure can range from 0.1 to 70 or even higher.

More detailed description of suitable polymerization conditions including examples of particle form, solution and fluidized bed polymerization arrangements are found in U.S. Patent Nos. 3,709,853 and 4,086,408.

Condensation of Alcohols

SSZ-95 can be used to condense lower aliphatic alcohols having 1 to 10 carbon atoms to a gasoline boiling point hydrocarbon product comprising mixed aliphatic and aromatic hydrocarbons. The process disclosed in U.S. Patent No. 3,894,107describes the typical conditions used in this process.

The catalyst can be in the hydrogen form or can be base-exchanged or impregnated to contain an ammonium or a metal cation component, typically in the range of from 0.05 to 5 wt. %. The metal cations that can be present include any of the metals of Groups 1-10 of the Periodic Table. However, in the case of Group 1 metals, the cation content should in no case be so large as to effectively inactivate the catalyst, nor should the exchange be such as to eliminate all acidity. There can be other processes involving treatment of oxygenated substrates where a basic catalyst is desired.

Partial Oxidation of Low Value Hydrocarbons

The partial oxidation of low value hydrocarbons such as alkanes and alkenes into high value products such as alcohols and epoxides is of great commercial interest. These oxidation products are not only valuable as is, but also as intermediates for specialty chemicals including pharmaceuticals and pesticides.

U.S. Patent No. 4,410,501 discloses a titanium-containing analogue of the all-silica ZSM-5 molecular sieve. This material (known as "TS-1") has been found to be useful in catalyzing a wide range of partial oxidation chemistries, for example the production of catechol and hydroquinone from phenol and hydrogen peroxide ($H_2O_2$) and the manufacture of propylene oxide and cyclohexanone oxime from propylene and cyclohexanone, respectively. In addition, TS-1 can be used to catalyze the reaction of alkanes and aqueous $H_2O_2$ to form alcohols and ketones (see, e.g., D.R.C. Huybrechts et al., Nature 1990, 345, 240-242; and T. Tatsumi et al., J. Chem. Soc. Chem. Commun. 1990, 476-477).

TS-1 has many salient features, other than its catalytic abilities, which make it attractive as a commercial catalyst. Most importantly, it is a solid. This allows for easy separation from the reactants and products (typically liquids) by simple, inexpensive filtration. Moreover, this solid has high thermal stability and a very long lifetime. Calcination in air at moderate temperatures (550°C) restores the material to its original catalytic ability. TS-1 performs best at mild

temperatures (<100°C) and pressures (101 kPa). The oxidant used for reactions catalyzed by TS-1 is aqueous $H_2O_2$, which is important because aqueous $H_2O_2$ is relatively inexpensive and its by-product is water. Hence, the choice of oxidant is favorable from both a commercial and environmental point of view.

While a catalyst system based on TS-1 has many useful features, it has one serious drawback. The zeolite structure of TS-1 includes a regular system of pores which are formed by nearly circular rings of ten silicon atoms (called 10-membered rings, or simply "10 rings") creating pore diameters of approximately 5.5 Å (0.55 nm). This small size results in the exclusion of molecules larger than 5.5 Å. Because the catalytically active sites are located within the pores of the zeolite, any exclusion of molecules from the pores results in poor catalytic activity.

SSZ-95 containing titanium oxide (Ti-SSZ-95) is useful as a catalyst in oxidation reactions, particularly in the oxidation of hydrocarbons. Examples of such reactions include the epoxidation of olefins, the oxidation of alkanes, and the oxidation of sulfur-containing, nitrogen-containing or phosphorus-containing compounds.

The amount of Ti-SSZ-95 catalyst employed is not critical, but should be sufficient so as to substantially accomplish the desired oxidation reaction in a practicably short period of time (i.e., a catalytically effective amount). The optimum quantity of catalyst will depend upon a number of factors including reaction temperature, the reactivity and concentration of the substrate, hydrogen peroxide concentration, type and concentration of organic solvent, as well as the activity of the catalyst. Typically, however, the amount of catalyst will be from 0.001 to 10 g/mol of substrate. Typically, the Ti-SSZ-95 is thermally treated (calcined) prior to use as a catalyst.

The oxidizing agent employed in the oxidation processes disclosed herein is a hydrogen peroxide source such as hydrogen peroxide or a hydrogen peroxide precursor (i.e., a compound which under the oxidation reaction conditions is capable of generating or liberating $H_2O_2$).

The amount of $H_2O_2$ relative to the amount of substrate is not critical, but must be sufficient to cause oxidation of at least some of the substrate. Typically, the molar ratio of $H_2O_2$ to substrate is from 100:1 to 1:100, e.g., from 10:1 to 1:10. When the substrate is an olefin containing more than one carbon-carbon double bond, additional hydrogen peroxide can be required. Theoretically, one equivalent of hydrogen peroxide is required to oxidize one equivalent of a mono-unsaturated substrate, but it can be desirable to employ an excess of one reactant to optimize selectivity to the epoxide. In particular, the use of a moderate to large excess (e.g., 50 to 200%) of olefin relative to $H_2O_2$ can be advantageous for certain substrates.

If desired, a solvent can additionally be present during the oxidation reaction in order to dissolve the reactants other than the Ti-SSZ-95, to provide better temperature control, or to favorably influence the oxidation rates and selectivities. The solvent, if present, can comprise from 1 to 99 wt. % of the total oxidation reaction mixture and is desirably selected such that it is a liquid at the oxidation reaction temperature. Organic compounds having boiling points at atmospheric pressure of from 50°C to 150°C are generally desirable for use. Excess hydrocarbon can serve as a solvent or diluent. Illustrative examples of other suitable solvents include ketones (e.g., acetone, methyl ethyl ketone, acetophenone), ethers (e.g., tetrahydrofuran, butyl ether), nitriles (e.g., acetonitrile), aliphatic and aromatic hydrocarbons, halogenated hydrocarbons, and alcohols (e.g., methanol, ethanol, isopropyl alcohol, tert-butyl alcohol, alpha-methyl benzyl alcohol, cyclohexanol). More than one type of solvent can be utilized. Water can also be employed as a solvent or diluent.

The reaction temperature is not critical, but should be sufficient to accomplish substantial conversion of the substrate within a reasonably short period of time. It is generally advantageous to carry out the reaction to achieve as high a hydrogen peroxide conversion as possible, typically at least 50%, e.g., at least 90% or at least 95%, consistent with reasonable selectivities. The optimum reaction temperature will be influenced by catalyst activity, substrate reactivity, reactant concentrations, and type of solvent employed, among other factors, but typically will be in a range of from 0°C to 150°C, e.g., from 25°C to 120°C. Reaction or residence times from one minute to 48 hours (e.g., from ten minutes to 8 hours) will typically be appropriate, depending upon the above-identified variables. Although sub-atmospheric pressures can be employed, the reaction is typically performed at atmospheric or at elevated pressure (typically, between 101 kPa and 10 MPa), especially when the boiling point of the substrate is below the oxidation reaction temperature. Generally, it is desirable to pressurize the reaction vessel sufficiently to maintain the reaction components as a liquid phase mixture. Most (over 50%) of the substrate should desirably be present in the liquid phase.

The oxidation process of this disclosure can be carried out in a batch, continuous, or semi-continuous manner using any appropriate type of reaction vessel or apparatus such as a fixed bed, transport bed, fluidized bed, stirred slurry, or CSTR reactor. The reactants can be combined all at once or sequentially. For example, the hydrogen peroxide or hydrogen peroxide precursor can be added incrementally to the reaction zone. The hydrogen peroxide could also be generated in situ within the same reactor zone where oxidation is taking place.

Once the oxidation has been carried out to the desired degree of conversion, the oxidized product can be separated and recovered from the reaction mixture using any appropriate technique such as fractional distillation, extractive distillation, liquid-liquid extraction, crystallization, or the like.

Olefin Epoxidation

One of the oxidation reactions for which Ti-SSZ-95 is useful as a catalyst is the epoxidation of olefins. The olefin substrate epoxidized in the process of this disclosure can be any organic compound having at least one ethylenically unsaturated functional group (i.e., a carbon-carbon double bond) and can be a cyclic, branched or straight-chain olefin. The olefin can contain aryl groups (e.g., phenyl, naphthyl). Typically, the olefin is aliphatic in character and contains from 2 to 20 carbon atoms. The use of light (low-boiling) $C_2$ to $C_{10}$ mono-olefins is especially advantageous.

More than one carbon-carbon double bond can be present in the olefin, i.e., dienes, trienes and other polyunsaturated substrates can be used. The double bond can be in a terminal or internal position in the olefin or can alternatively form part of a cyclic structure (as in cyclooctene, for example). Other examples of suitable substrates include unsaturated fatty acids or fatty acid derivatives such as esters.

The olefin can contain substituents other than hydrocarbon substituents such as halide, carboxylic acid, ether, hydroxy, thiol, nitro, cyano, ketone, acyl, ester, anhydride, amino, and the like.

Exemplary olefins suitable for use in the process of this disclosure include ethylene, propylene, the butenes (i.e., 1,2-butene, 2,3-butene, isobutylene), butadiene, the pentenes, isoprene, 1-hexene, 3-hexene, 1-heptene, 1-octene, diisobutylene, 1-nonene, 1-tetradecene, pentamyrcene, camphene, 1-undecene, 1-dodecene, 1-tridecene, 1-tetradecene, 1-pentadecene, 1-hexadecene, 1-heptadecene, 1-octadecene, 1-nonadecene, 1-eicosene, the trimers and tetramers of propylene, cyclopentene, cyclohexene, cycloheptene, cyclooctene, cyclooctadiene, dicyclopentadiene, methylenecyclopropane, methylenecyclopentane, methylenecyclohexane, vinyl cyclohexane, vinyl cyclohexene, allyl chloride, the dichlorobutenes, allyl alcohol, allyl carbonate, allyl acetate, alkyl acrylates and methacrylates, diallyl maleate, diallyl phthalate, and unsaturated fatty acids, such as oleic acid, linolenic acid, linoleic acid, erucic acid, palmitoleic acid, and ricinoleic acid and their esters (including mono-, di-, and triglyceride esters) and the like.

Olefins which are especially useful for epoxidation are the $C_2$ to $C_{20}$ olefins having the general structure (1):

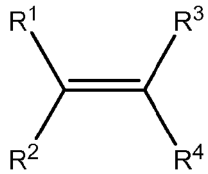

(1)

wherein $R^1$, $R^2$, $R^3$ and $R^4$ are the same or different and are selected from the group consisting of hydrogen and $C_1$ to $C_{18}$ alkyl.

Mixtures of olefins can be epoxidized and the resulting mixtures of epoxides either employed in the mixed form or separated into the different component epoxides.

The present disclosure further provides a process for oxidation of hydrocarbons comprising contacting the hydrocarbon with hydrogen peroxide in the presence of a catalytically effective amount of Ti-SSZ-95 for a time and at a temperature effective to oxidize the hydrocarbon.

Oxygenate Conversion

The disclosed herein comprises a process for catalytic conversion of a feedstock comprising one or more oxygenates comprising alcohols and ethers to a hydrocarbon product containing light olefins, i.e., $C_2$, $C_3$ and/or $C_4$ olefins. The feedstock is contacted with SSZ-95 at effective process conditions to produce light olefins. The term "oxygenate" as used herein designates compounds such as alcohols, ethers, and carbonyl compounds (e.g., aldehydes, ketones, carboxylic acids). The oxygenate can contain from 1 to 10 carbon atoms, e.g., from 1 to 4 carbon atoms. The representative oxygenates include lower straight chained branched alcohols, and their unsaturated counterparts. Particularly suitable oxygenate compounds are methanol, dimethyl ether, and mixtures thereof

The process disclosed can be conducted in the presence of one or more diluents which can be present in the oxygenate feed in an amount of from 1 to 99 mole %, based on the total number of moles of all feed and diluent components. Diluents include helium, argon, nitrogen, carbon monoxide, carbon dioxide, hydrogen, water, paraffins, hydrocarbons (such as methane and the like), aromatic compounds, or mixtures thereof. U. S. Patent Nos. 4,677,242; 4,861,938; and 4,677,242 emphasize the use of a diluent to maintain catalyst selectivity toward the production of light olefins, particularly ethylene.

The oxygenate conversion is desirably conducted in the vapor phase such that the oxygenate feedstock is contacted in a vapor phase in a reaction zone with SSZ-95 at effective process conditions to produce hydrocarbons, i.e., an effective temperature, pressure, WHSV and, optionally, an effective amount of diluent. The process is conducted for a period of time sufficient to produce the desired light olefins. In general, the residence time employed to produce

the desired product can vary from seconds to a number of hours. It will be readily appreciated that the residence time will be determined to a significant extent by the reaction temperature, the molecular sieve catalyst, the WHSV, the phase (liquid or vapor) and process design characteristics. The oxygenate feedstock flow rate affects olefin production. Increasing the feedstock flow rate increases WHSV and enhances the formation of olefin production relative to paraffin production. However, the enhanced olefin production relative to paraffin production is offset by a diminished conversion of oxygenate to hydrocarbons.

Light olefin products will form, although not necessarily in optimum amounts, at a wide range of pressures, including but not limited to autogenous pressures and pressures in the range from 0.1 kPa to 10 MPa. Conveniently, the pressure can be in the range from 7 kPa to 5 MPa, e.g., from 50 kPa to 1 MPa. The foregoing pressures are exclusive of diluents, if any are present, and refer to the partial pressure of the feedstock as it relates to oxygenate compounds and/or mixtures thereof. Lower and upper extremes of pressure can adversely affect selectivity, conversion, coking rate, and/or reaction rate; however, light olefins such as ethylene and/or propylene still may form.

The temperature which can be employed in the oxygenate conversion process can vary over a wide range depending, at least in part, on the molecular sieve catalyst. In general, the process can be conducted at an effective temperature of from 200°C to 700°C. At the lower ends of the temperature range, and thus generally at a lower rate of reaction, the formation of the desired light olefins can become low. At the upper ends of the range, the process cannot form an optimum amount of light olefins and catalyst deactivation can be rapid.

The molecular sieve catalyst can be incorporated into solid particles in which the catalyst is present in an amount effective to promote the desired conversion of oxygenates to light olefins. In one aspect, the solid particles comprise a catalytically effective amount of the catalyst and at least one matrix material selected from the group consisting of binder materials, filler materials and mixtures thereof to provide a desired property or properties, e.g., desired catalyst dilution, mechanical strength and the like to the solid particles. Such matrix materials are often, to some extent, porous in nature and can or cannot be effective to promote the desired reaction. Filler and binder materials include, for example, synthetic and naturally occurring substances such as metal oxides, clays, silicas, aluminas, silica-aluminas, silica-magnesias, silica-zirconias, silicathorias and the like. If matrix materials are included in the catalyst composition, the molecular sieve desirably comprises from 1 to 99 wt. % (e.g., from 5 to 90 wt. % or from 10 to 80 wt. %) of the total composition.

## Acvlation

SSZ-95 can be used in a catalyst for acylating an aromatic substrate $ArH_k$, where k is at least 1, by reacting the aromatic substrate with an acylating agent in the presence of the catalyst. The product of the acylation reaction is $ArH_{k-1}COR^5$ where $R^5$ is an organic radical.

Examples of the aromatic substrate include, but are not limited to, benzene, toluene, anisole and 2-naphthol. Examples of the acylating agent included, but are not limited to, carboxylic acid derivatives, carboxylic acids, acid anhydrides, esters, and acyl halides.

Reaction conditions are known in the art (see, e.g., U. S. Patent Nos. 6,459,000; 6,548,722; and 6,630,606). Typically, the acylation reaction is conducted with a weight ratio of the catalyst to the acylating agent of from 0.03 to 0.5, a mole ratio of aromatic substrate to acylating agent of from 1.0 to 20, a reaction temperature of from 20°C to 200°C, a reaction pressure of from 101 to 507 kPa, and a reaction time of from 0.05 to 20 h.

## Synthesis of Amines

SSZ-95 can be used in a catalyst to prepare methylamine or dimethylamine. Dimethylamine is generally prepared in industrial quantities by continuous reaction of methanol (and/or dimethyl ether) and ammonia in the presence of a silica-alumina catalyst. The reactants are typically combined in the vapor phase, at temperatures of from 300°C to 500°C, and at elevated pressures. Such a process is disclosed in U. S. Patent No. 4,737,592.

The catalyst is used in its acid form. Acid forms of molecular sieves can be prepared by a variety of techniques. Desirably, the molecular sieve used to prepare dimethylamine will be in the hydrogen form, or have an alkali or alkaline earth metal, such as Na, K, Rb, or Cs, ion-exchanged into it.

The process disclosed herein involves reacting methanol, dimethyl ether, or a mixture thereof and ammonia in amounts sufficient to provide a carbon/nitrogen (C/N) ratio of from 0.2 to 1.5, e.g., from 0.5 to 1.2. The reaction is conducted at a temperature of from 250°C to 450°C, e.g., from 300°C to 400°C. Reaction pressures can vary from 7 to 7000 kPa, e.g., from 70 to 3000 kPa. A methanol and/or dimethyl ether space time of from 0.01 to 80 $h^{-1}$ (e.g., from 0.10 to 1.5 $h^{-1}$) is typically used. This space time is calculated as the mass of catalyst divided by the mass flow rate of methanol/dimethyl ether introduced into the reactor.

Beckmann Rearrangement

SSZ-95 can be used as a catalyst in the transformation of oximes (such as cyclohexanone oxime) to amides (such as epsilon-caprolactam), also known as a Beckmann rearrangement. The Beckmann rearrangement is shown below (where sulfuric acid is used instead of a molecular sieve catalyst).

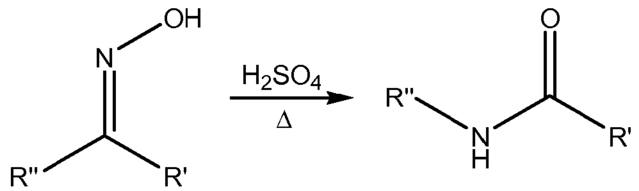


Amides, and in particular epsilon-caprolactam, are known in literature as important intermediates for chemical syntheses and as raw materials for the preparation of polyamide resins.

Caprolactam is produced industrially by cyclohexanone oxime rearrangement in liquid phase using sulfuric acid or oleum. The rearranged product is neutralized with ammonia causing the joint formation of ammonium sulfate. This technology has numerous problems linked to the use of sulfuric acid, to the formation of high quantities of ammonium sulfate, with relative problems of disposal, corrosion of the equipment owing to the presence of acid vapors, etc.

Alternative processes have been proposed in the literature for the catalytic rearrangement of cyclohexanone oxime into caprolactam, in which solids of an acid nature are used, as catalysts, selected from derivatives of boric acid, zeolites, non-zeolitic molecular sieves, solid phosphoric acid, mixed metal oxides, etc.

In particular, European Patent No. 234,088 describes a method for preparing caprolactam which comprises putting cyclohexanone oxime in gaseous state in contact with aluminosilicate zeolites such as ZSM-5, ZSM-11 or ZSM-23 having a constraint index of between 1 and 12, a SiO2/$Al_2O_3$ mole ratio of at least 500 and an external acid functionality of less than 5 micro-equivalents/g.

With the aim of providing another method for the preparation of amides, and in particular of caprolactam, a new process has now been found which uses a catalyst comprising SSZ-95. The present disclosure therefore relates to a process for the preparation of amides via the catalytic rearrangement of oximes which comprises putting an oxime in the vapor phase in contact with a catalyst comprising a crystalline molecular sieve having a mole ratio of at least 10 of (1) at least one oxide of at least one tetravalent element to (2) one or more oxides selected from the group consisting of oxides of trivalent elements, pentavalent elements, and mixtures thereof, and having, in its calcined from, the X-ray diffraction lines of Table 6. The molecular sieve can have a mole ratio of at least 10 of (1) silicon oxide to (2) an oxide selected from boron oxide, aluminum oxide, gallium oxide, indium oxide, and mixtures thereof.

Other methods for converting oximes to amides via Beckmann rearrangement are disclosed in U.S. Patent Nos. 4,883,915 and 5,942,613.

A desirable amide is epsilon-caprolactam (ε-caprolactam) and the desirable oxime is cyclohexanone oxime. In particular, the catalytic rearrangement of the cyclohexanone oxime takes place at a pressure of from 5 kPa to 1 MPa and at a temperature of from 250°C and 500°C, e.g., from 300°C to 450°C. More specifically, the cyclohexanone oxime, in vapor phase, is fed to the reactor containing the catalyst in the presence of a solvent and optionally an incondensable gas. The cyclohexanone oxime is dissolved in the solvent and the mixture thus obtained is then vaporized and fed to the reactor. The solvent should be essentially inert to the oxime and the amide, as well as the catalyst. Useful solvents include, but are not limited to, lower boiling hydrocarbons, alcohols and ethers.

Desirable solvents are of the type $R^5$-O-$R^6$ wherein $R^5$ is a $C_1$ to $C_4$ alkyl chain and $R^6$ can be a hydrogen atom or an alkyl chain containing a number of carbon atoms less than or equal to $R^5$. These solvents can be used alone or mixed with each other or combined with an aromatic hydrocarbon such as benzene or toluene. Alcohols with a $C_1$ to $C_2$ alkyl chain are particularly desirable.

The cyclohexanone oxime is fed to the rearrangement reactor with a weight ratio with respect to the catalyst which is such as to give a WHSV, expressed as kg of cyclohexanone oxime/kg of catalyst/time, of from 0.1 to 50 $h^{-1}$, e.g., from 0.5 to 20 $h^{-1}$.

The deterioration of the catalyst is due to the formation of organic residues which obstruct the pores of the catalyst and poison its active sites. The deterioration process is slow and depends on the operating conditions and in particular the space velocity, solvent, temperature, composition of the feeding. The catalytic activity however can be efficiently reintegrated by the combustion of the residues, by treatment in a stream of air and nitrogen at a temperature of from 450°C to 600°C.

Gas Separation

SSZ-95 can be used to separate gasses. For example, it can be used to separate carbon dioxide from natural gas. Typically, the molecular sieve is used as a component in a membrane that is used to separate the gases. Examples of such membranes are disclosed in U. S. Patent No. 6,508,860.

Treatment of Engine Exhaust (Cold Start Emissions)

Gaseous waste products resulting from the combustion of hydrocarbon fuels, such as gasoline and fuel oils, comprise carbon monoxide, hydrocarbons and nitrogen oxides as products of combustion or incomplete combustion, and can pose a serious health problem with respect to pollution of the atmosphere. While exhaust gases from other carbonaceous fuel-burning sources, such as stationary engines, industrial furnaces, etc., contribute substantially to air pollution, the exhaust gases from automotive engines are a principal source of pollution. Because of these concerns, the U.S. Environmental Protection Agency has promulgated strict controls on the amounts of carbon monoxide, hydrocarbons and nitrogen oxides which automobiles can emit. The implementation of these controls has resulted in the use of catalytic converters to reduce the amount of pollutants emitted from automobiles.

In order to achieve the simultaneous conversion of carbon monoxide, hydrocarbon and nitrogen oxide pollutants, it has become the practice to employ catalysts in conjunction with air-to-fuel ratio control means which functions in response to a feedback signal from an oxygen sensor in the engine exhaust system. Although these three component control catalysts work quite well after they have reached operating temperature of about 300°C, at lower temperatures they are not able to convert substantial amounts of the pollutants. What this means is that when an engine and in particular an automobile engine is started up, the three component control catalyst is not able to convert the hydrocarbons and other pollutants to innocuous compounds.

Adsorbent beds have been used to adsorb the hydrocarbons during the cold start portion of the engine. Although the process typically will be used with hydrocarbon fuels, the present disclosure can also be used to treat exhaust streams from alcohol-fueled engines. The adsorbent bed is typically placed immediately before the catalyst. Thus, the exhaust stream is first flowed through the adsorbent bed and then through the catalyst. The adsorbent bed preferentially adsorbs hydrocarbons over water under the conditions present in the exhaust stream. After a certain amount of time, the adsorbent bed has reached a temperature (typically about 150°C) at which the bed is no longer able to remove hydrocarbons from the exhaust stream. That is, hydrocarbons are actually desorbed from the adsorbent bed instead of being adsorbed. This regenerates the adsorbent bed so that it can adsorb hydrocarbons during a subsequent cold start. The use of adsorbent beds to minimize hydrocarbon emissions during a cold start engine operation is known in the art. See, for example, U.S. Patent Nos. 2,942,932; 3,699,683; and 5,078,979.

As stated, this disclosure generally relates to a process for treating an engine exhaust stream and, in particular, to a process for minimizing emissions during the cold start operation of an engine. The engine consists of any internal or external combustion engine which generates an exhaust gas stream containing noxious components or pollutants including unburned or thermally degraded hydrocarbons or similar organics. Other noxious components usually present in the exhaust gas include nitrogen oxides and carbon monoxide. The engine can be fueled by a hydrocarbon fuel. As used herein, the term "hydrocarbon fuel" includes hydrocarbons, alcohols and mixtures thereof. Examples of hydrocarbons which can be used to fuel the engine are the mixtures of hydrocarbons which make up gasoline or diesel fuel. The alcohols which can be used to fuel engines include ethanol and methanol. Mixtures of alcohols and mixtures of alcohols and hydrocarbons can also be used. The engine can be a jet engine, gas turbine, internal combustion engine, such as an automobile, truck or bus engine, a diesel engine or the like. The process of this disclosure is particularly suited for an internal combustion engine mounted in an automobile.

When the engine is started up, it produces a relatively high concentration of hydrocarbons in the engine exhaust gas stream as well as other pollutants. Pollutants will be used herein to collectively refer to any unburned fuel components and combustion byproducts found in the exhaust stream. For example, when the fuel is a hydrocarbon fuel, hydrocarbons, nitrogen oxides, carbon monoxide and other combustion byproducts will be found in the engine exhaust gas stream. The temperature of this engine exhaust stream is relatively cool, generally below 500°C and typically in the range of from 200°C to 400°C. This engine exhaust stream has the above characteristics during the initial period of engine operation, typically for the first 30 to 120 seconds after startup of a cold engine. The engine exhaust stream will typically contain from 500 to 1000 ppm hydrocarbons by volume.

In one embodiment, the engine exhaust gas stream which is to be treated is flowed over a combination of molecular sieves which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere. The combination of molecular sieves includes SSZ-95 in combination with: (1) a small pore crystalline molecular sieve or mixture of molecular sieves having pores no larger than 8-membered rings selected from

the group consisting of SSZ-13, SSZ-16, SSZ-36, SSZ-39. SSZ-50, SSZ-52 and SSZ-73 and having a mote ratio of at least 10 of (a) at least one oxide of at least one tetravalent element to (b) one or more oxides selected from the group consisting of oxides of trivalent elements, pentavalent elements, and mixtures thereof; and/or (2) a large pore crystalline molecular sieve having pores at least as large as 10-membered rings selected from the group consisting of SSZ-26, SSZ-33, SSZ-64, zeolite Beta, CIT-1 , CIT-6 and ITQ-4 and having a mole ratio of at least 10 of (a) at least one oxide of at least one tetravalent element to (b) one or more oxides selected from the group consisting of oxides of trivalent elements, pentavalent elements, and mixtures thereof.

The engine exhaust gas stream which is to be treated is flowed over a molecular sieve bed comprising molecular sieve SSZ-95 as a first exhaust stream. The first exhaust stream which is discharged from the molecular sieve bed is now flowed over a catalyst to convert the pollutants contained in the first exhaust stream to innocuous components and provide a treated exhaust stream which is discharged into the atmosphere. It is understood that prior to discharge into the atmosphere, the treated exhaust stream can be flowed through a muffler or other sound reduction apparatus well known in the art.

The catalyst which is used to convert the pollutants to innocuous components is usually referred to in the art as a three-component control catalyst because it can simultaneously oxidize any residual hydrocarbons present in the first exhaust stream to carbon dioxide and water, oxidize any residual carbon monoxide to carbon dioxide and reduce any residual nitric oxide to nitrogen and oxygen. In some cases the catalyst cannot be required to convert nitric oxide to nitrogen and oxygen, e.g., when an alcohol is used as the fuel. In this case the catalyst is called an oxidation catalyst. Because of the relatively low temperature of the engine exhaust stream and the first exhaust stream, this catalyst does not function at a very high efficiency, thereby necessitating the molecular sieve bed.

When the molecular sieve bed reaches a sufficient temperature, typically from 150°C to 200°C, the pollutants which are adsorbed in the bed begin to desorb and are carried by the first exhaust stream over the catalyst. At this point the catalyst has reached its operating temperature and is therefore capable of fully converting the pollutants to innocuous components.

The adsorbent bed used in this disclosure can be conveniently employed in particulate form or the adsorbent can be deposited onto a solid monolithic carrier. When particulate form is desired, the adsorbent can be formed into shapes such as pills, pellets, granules, rings, spheres, etc. In the employment of a monolithic form, it is usually most convenient to employ the adsorbent as a thin film or coating deposited on an inert carrier material which provides the structural support for the adsorbent. The inert carrier material can be any refractory material such as ceramic or metallic materials. It is desirable that the carrier material be unreactive with the adsorbent and not be degraded by the gas to which it is exposed. Examples of suitable ceramic materials include sillimanite, petalite, cordierite, mullite, zircon, zircon mullite, spondumene, alumina-titanate, etc. Additionally, metallic materials which are within the scope of this disclosure include metals and alloys as disclosed in U.S. Patent No. 3,920,583 which are oxidation resistant and are otherwise capable of withstanding high temperatures.

The carrier material can best be utilized in any rigid unitary configuration which provides a plurality of pores or channels extending in the direction of gas flow. The configuration can be a honeycomb configuration. The honeycomb structure can be used advantageously in either unitary form, or as an arrangement of multiple modules. The honeycomb structure is usually oriented such that gas flow is generally in the same direction as the cells or channels of the honeycomb structure. For a more detailed discussion of monolithic structures, refer to U.S. Patent Nos. 3,767,453 and 3,785,998.

The molecular sieve is deposited onto the carrier by any convenient way well known in the art. A desirable method involves preparing a slurry using the molecular sieve and coating the monolithic honeycomb carrier with the slurry. The slurry can be prepared by means known in the art such as combining the appropriate amount of the molecular sieve and a binder with water. This mixture is then blended by using means such as sonication, milling, etc. This slurry is used to coat a monolithic honeycomb by dipping the honeycomb into the slurry, removing the excess slurry by draining or blowing out the channels, and heating to about 100°C. If the desired loading of molecular sieve is not achieved, the above process can be repeated as many times as required to achieve the desired loading.

Instead of depositing the molecular sieve onto a monolithic honeycomb structure, the molecular sieve can be formed into a monolithic honeycomb structure by means known in the art.

The adsorbent can optionally contain one or more catalytic metals dispersed thereon. The metals which can be dispersed on the adsorbent are the noble metals which consist of platinum, palladium, rhodium, ruthenium, and mixtures thereof. The desired noble metal can be deposited onto the adsorbent, which acts as a support, in any suitable manner well known in the art. One example of a method of dispersing the noble metal onto the adsorbent support involves impregnating the adsorbent support with an aqueous solution of a decomposable compound of the desired noble metal or metals, drying the adsorbent which has the noble metal compound dispersed on it and then calcining in air at a temperature of 400°C to 500°C for a time of from 1 to 4 hours. By decomposable compound is meant a compound which upon heating in air gives the metal or metal oxide. Examples of the decomposable compounds which can be used are set forth in U.S. Patent No. 4,791,091. Examples of decomposable compounds are chloroplatinic acid, rhodium trichloride, chloropalladic acid, hexachloroiridate(IV) acid and hexachlororuthenate(IV). It is typical that the noble metal be present

in an amount ranging from 0.01 to 4 wt. % of the adsorbent support. Specifically, in the case of platinum and palladium the range is from 0.1 to 4 wt. %, while in the case of rhodium and ruthenium the range is from 0.01 to 2 wt. %.

These catalytic metals are capable of oxidizing the hydrocarbon and carbon monoxide and reducing the nitric oxide components to innocuous products. Accordingly, the adsorbent bed can act both as an adsorbent and as a catalyst.

The catalyst which is used in this disclosure is selected from any three component control or oxidation catalyst well known in the art. Examples of catalysts are those described in U.S. Patent Nos. 4,528,279; 4,760,044; 4,791,091; 4,868,148; and 4,868,149. Desirable catalysts well known in the art are those that contain platinum and rhodium and optionally palladium, while oxidation catalysts usually do not contain rhodium. Oxidation catalysts usually contain platinum and/or palladium metal. These catalysts can also contain promoters and stabilizers such as barium, cerium, lanthanum, nickel, and iron. The noble metals promoters and stabilizers are usually deposited on a support such as alumina, silica, titania, zirconia, alumino silicates, and mixtures thereof with alumina being desirable. The catalyst can be conveniently employed in particulate form or the catalytic composite can be deposited on a solid monolithic carrier with a monolithic carrier being desirable. The particulate form and monolithic form of the catalyst are prepared as described for the adsorbent above. The molecular sieve used in the adsorbent bed is SSZ-95.

Reduction of Oxides of Nitrogen

SSZ-95 can be used for the catalytic reduction of the oxides of nitrogen in a gas stream. Typically, the gas stream also contains oxygen, often a stoichiometric excess thereof. Also, the molecular sieve can contain a metal or metal ions within or on it which are capable of catalyzing the reduction of the nitrogen oxides. Examples of such metals or metal ions include lanthanum, chromium, manganese, iron, cobalt, rhodium, nickel, palladium, platinum, copper, zinc, and mixtures thereof.

One example of such a process for the catalytic reduction of oxides of nitrogen in the presence of a zeolite is disclosed in U.S. Patent No. 4,297,328. There, the catalytic process is the combustion of carbon monoxide and hydrocarbons and the catalytic reduction of the oxides of nitrogen contained in a gas stream, such as the exhaust gas from an internal combustion engine. The zeolite used is metal ion-exchanged, doped or loaded sufficiently so as to provide an effective amount of catalytic copper metal or copper ions within or on the zeolite. In addition, the process is conducted in an excess of oxidant, e.g., oxygen.

## EXAMPLES

The following illustrative examples are intended to be non-limiting.

As-synthesized organic template-containing SSZ-32x used as the starting material for the preparation of all samples described in the following examples were prepared according to the method disclosed in US 8,545,805 to Zones et al., granted on October 1, 2013. The starting as-synthesized SSZ-32x was dried at a temperature of between 95°C and 200°C, a temperature sufficient to dehydrate the product by removing all liquid water and water retained within the intercrystalline pores of the molecular sieve.

### EFFECT OF PRE-CALCINING TEMPERATURE ON MICROPORE VOLUME

In Examples 1 through 7 below, samples of SSZ-32x were pre-calcined at various temperatures between 200 and 400°C prior to undergoing ammonium ion-exchange. In Comparative Example 7, SSZ-32x was pre-calcined at a conventional calcination temperature, namely 595°C. As will be shown below, by maintaining a lower pre-calcination temperature prior to the ammonium ion-exchange step (during the pre-calcination step), it was surprising to find the isomerization selectivity of the molecular sieve was enhanced as will be shown in Examples 8 through 13.

### EXAMPLE 1

3.38g of as-synthesized SSZ-32x was pre-calcined in a muffle furnace under an atmosphere of dry air at a heating rate of 1°C/minute (min.) to 120°C and held for 120 min., followed by a second ramp of 1°C/min. to 300°C and held at this temperature for 180 min. Finally, the sample was cooled down to 150°C. The weight loss was 5.02 wt%.

The pre-calcined sample was then exchanged into the ammonium form as follows. An amount of ammonium nitrate equal to the mass of the sample to be exchanged was fully dissolved in an amount of deionized water ten times the mass of the sample. The sample was then added to the ammonium nitrate solution and the suspension was sealed in a flask and heated in an oven at 95°C overnight. The flask was removed from the oven, and the sample was recovered immediately by filtration. This ammonium exchange procedure was repeated on the recovered sample, washed with copious amount of deionized water to a conductivity of less than 50 pS/cm and finally dried in an oven at 120°C for three hours.

The ammonium-exchanged sample was calcined at 400°C, and then a micropore analysis was conducted. The sample had a micropore volume of 0.013 cc/g, external surface area of 232.9 $m^2$/g and a BET surface area of 267.3 $m^2$/g.

**EXAMPLE 2**

4.24g of as-synthesized SSZ-32x was pre-calcined in a muffle furnace under an atmosphere of dry air at a heating rate of 1°C/min. to 120°C and held for 120 min. followed by a second ramp of 1°C/min. to 320°C and held at this temperature for 180 min. Finally, the sample was cooled down to 150°C. The weight loss was 5.17 wt%. The pre-calcined sample was then exchanged into the ammonium form following the procedure described in Example 1. Sample was washed with copious amount of deionized water to a conductivity less than 50 pS/cm and finally dried in an oven at 120°C for three hours.

The ammonium-exchanged sample was calcined at 400°C, and then a micropore analysis was conducted. The sample had a micropore volume of 0.0133 cc/g, external surface area of 237.1 $m^2$/g and BET surface area of 272.0 $m^2$/g.

**EXAMPLE 3**

4.39g of as-synthesized SSZ-32x was pre-calcined in a muffle furnace under an atmosphere of dry air at a heating rate of 1°C/min. to 120°C and held for 120 min. followed by a second ramp of 1°C/min. to 350°C and held at this temperature for 180 min. Finally, the sample was cooled down to 150°C. The weight loss was 5.72 wt%. The pre-calcined sample was then exchanged into the ammonium form following the procedure described in Example 1. Sample was washed with copious amount of deionized water to a conductivity less than 50 pS/cm and finally dried in an oven at 120°C for three hours.

The ammonium-exchanged sample was calcined at 400°C, and then a micropore analysis was conducted. The sample had a micropore volume of 0.0135 cc/g, external surface area of 231.4 $m^2$/g and BET surface area of 266.6 $m^2$/g.

**EXAMPLE 4**

4.41 g of as-synthesized SSZ-32x was pre-calcined in a muffle furnace under an atmosphere of dry air at a heating rate of 1°C/min. to 120°C and held for 120 min. followed by a second ramp of 1°C/min. to 400°C and held at this temperature for 180 min. Finally, the sample was cooled down to 150°C. The weight loss was 8.23 wt%. The pre-calcined sample was then exchanged into the ammonium form following the procedure described in Example 1. Sample was washed with copious amount of deionized water to a conductivity less than 50 pS/cm and finally dried in an oven at 120°C for three hours.

The ammonium-exchanged sample was calcined at 400°C, and then a micropore analysis was conducted. The sample had a micropore volume of 0.0141 cc/g, external surface area of 229.8 $m^2$/g and BET surface area of 266.4 $m^2$/g.

**EXAMPLE 5**

8.51g of as-synthesized SSZ-32x was pre-calcined in a muffle furnace under an atmosphere of dry air at a heating rate of 1°C/min. to 120°C and held for 120 min. followed by a second ramp of 1°C/min. to 200°C and held at this temperature for 180 min. Finally, the sample was cooled down to 150°C. The weight loss was 4.36 wt%. The pre-calcined sample was then exchanged into the ammonium form following the procedure described in Example 1. Sample was washed with copious amount of deionized water to a conductivity less than 50 pS/cm and finally dried in an oven at 120°C for three hours.

**EXAMPLE 6**

A 9.09g of as-synthesized SSZ-32x sample was pre-calcined in a muffle furnace under an atmosphere of dry air at a heating rate of 1°C/min. to 120°C and held for 120 min. followed by a second ramp of 1°C/min. to 250°C and held at this temperature for 180 min. Finally, the sample was cooled down to 150°C. The weight loss was 4.72 wt%. The pre-calcined sample was then exchanged into the ammonium form following the procedure described in Example 1. Sample was washed with copious amount of deionized water to a conductivity less than 50 pS/cm and finally dried in an oven at 120°C for three hours.

**EXAMPLE 7 (Comparative)**

The as-synthesized SSZ-32x sample was converted into the potassium form under an atmosphere of dry air at a heating rate of 1°C/min. to 120°C and held for 120 min followed by a second ramp of 1°C/min. to 540°C and held

at this temperature for 180 min and lastly a third ramp of 1°C/min. to 595°C and held at this temperature for 180 min. Finally, the sample was cooled down to 120°C. The total weight loss was 12.23 wt% (may contain some residual water). The calcined sample was then exchanged into the ammonium form following the procedure described in Example 1. Sample was washed with copious amount of deionized water to a conductivity less than 50 μS/cm and finally dried in an oven at 120°C for three hours.

The ammonium-exchanged sample above was calcined at 400°C, and then a micropore analysis was conducted. The sample had a micropore volume of 0.0414 cc/g, external surface area of 235.9 $m^2/g$ and BET surface area of 330.8 $m^2/g$.

## EFFECT OF PRE-CALCINING TEMPERATURE ON ISOMERIZATION SELECTIVITY USING PALLADIUM-CONTAINING CATALYST

### EXAMPLE 8

Palladium ion-exchange was carried out on the ammonium-exchanged sample from Example 1 using palladiumtetraamine dinitrate (0.5 wt% Pd). This palladium-exchanged sample was dried at 95°C and then calcined in air at 482°C for 3 hours to convert the palladiumtetraamine dinitrate to palladium oxide.

0.5 g of this Pd-exchanged sample was loaded in the center of a 23 inch-long by 0.25 inch outside diameter stainless steel reactor tube with alundum loaded upstream of the catalyst for preheating the feed (total pressure of 1200 psig; down-flow hydrogen rate of 160 mL/min (when measured at 1 atmosphere pressure and 25°C.); down-flow liquid feed rate=1 mL/hour.). All materials were first reduced in flowing hydrogen at about 315°C for 1 hour. Products were analyzed by on-line capillary gas chromatography (GC) once every thirty minutes. Raw data from the GC was collected by an automated data collection/processing system and hydrocarbon conversions were calculated from the raw data.

The catalyst was tested at about 260°C initially to determine the temperature range for the next set of measurements. The overall temperature range will provide a wide range of hexadecane conversion with the maximum conversion just below and greater than 96%. At least five on-line GC injections were collected at each temperature. Conversion was defined as the amount of hexadecane reacted to produce other products (including iso-$C_{16}$). Yields were expressed as weight percent of products other than n-$C_{16}$ and included iso-$C_{16}$ isomers as a yield product. The results are shown in Table 1.

TABLE 1

| | |
|---|---|
| Pre-calcination Temperature (°C) | 300 |
| Weight Loss after pre-calcination (wt.%) | 5.02 |
| Micropore Vol. (cc/g) | 0.013 |
| Ext. area ($m^2/g$) | 232.9 |
| BET area ($m^2/g$) | 267.3 |
| Isomerization Selectivity at 96% Conversion | 83% |
| Temperature at 96% Conversion (°F) | 534 |
| $C_4$- Cracking (%) | 2.0 |

### EXAMPLE 9

Palladium ion-exchange was carried out on the ammonium-exchanged sample from Example 2 per the teachings of Example 8. The palladium-exchanged sample was tested for the selective hydroconversion of n-hexadecane under the conditions described in Example 8. The results are presented in Table 2.

The isomerization selectivity at 96% conversion for this sample is better than those produced by prior art and conventional methods for the conversion of the n-C16 feed to isomerized products, with lower gas make due to n-C16 cracking.

TABLE 2

| | |
|---|---|
| Pre-calcination Temperature (°C) | 320 |
| Weight Loss after pre-calcination (wt.%) | 5.17 |
| Micropore Vol. (cc/g) | 0.0133 |
| Ext. area ($m^2/g$) | 237.1 |
| BET area ($m^2/g$) | 272.0 |

(continued)

| | |
|---|---|
| Activation temperature (°C) | 482 |
| Isomerization Selectivity at 96% Conversion | 83.2% |
| Temperature (°F) | 532 |
| $C_4$- Cracking (%) | 1.90 |

**EXAMPLE 10**

Palladium ion-exchange was carried out on the ammonium-exchanged sample from Example 3 per the teachings of Example 8. The palladium-exchanged sample was tested for the selective hydroconversion of n-hexadecane under the conditions described in Example 8. The results are shown in Table 3. The isomerization selectivity at 96% conversion for this sample is better than those produced by prior art and conventional methods for the conversion of the n-$C_{16}$ feed to isomerized products, with lower gas make due to n-$C_{16}$ cracking.

TABLE 3

| | |
|---|---|
| Pre-calcination Temperature (°C) | 350 |
| Weight Loss after pre-calcination (wt.%) | 5.72 |
| Micropore Vol. (cc/g) | 0.0135 |
| Ext. area ($m^2$/g) | 231.4 |
| BET area ($m^2$/g) | 266.6 |
| Activation temperature (°C) | 482 |
| Isomerization Selectivity at 96% Conversion | 84.4% |
| Temperature (°F) | 532 |
| $C_4$- Cracking (%) | 1.80 |

**EXAMPLE 11**

Palladium ion-exchange was carried out on the ammonium-exchanged sample from Example 4 per the teachings of Example 8. The palladium-exchanged sample was tested for the selective hydroconversion of n-hexadecane under the conditions described in Example 8. The results are presented in Table 4. The isomerization selectivity at 96% conversion for this sample is better than those produced by prior art and conventional methods for the conversion of the n-$C_{16}$ feed to isomerized products, with lower gas make due to n-$C_{16}$ cracking.

TABLE 4

| | |
|---|---|
| Pre-calcination Temperature (°C) | 400 |
| Weight Loss after pre-calcination (wt.%) | 8.23% |
| Micropore Vol. (cc/g) | 0.0141 |
| Ext. area ($m^2$/g) | 229.8 |
| BET area ($m^2$/g) | 266.4 |
| Activation temperature (°C) | 482 |
| Isomerization Selectivity at 96% Conversion | 83.4% |
| Temperature (°F) | 531 |
| $C_4$- Cracking (%) | 2.05% |

**EXAMPLE 12**

Palladium ion-exchange was carried out on the ammonium-exchanged sample from Example 5 per the teachings of Example 8. The palladium-exchanged sample was tested for the selective hydroconversion of n-hexadecane under the conditions described in Example 8. The results are presented in Table 5. The isomerization selectivity at 96% conversion for this sample is better than those produced by prior art and conventional methods for the conversion of the n-$C_{16}$ feed to isomerized products, with lower gas make due to n-$C_{16}$ cracking.

TABLE 5

| | |
|---|---|
| Pre-calcination Temperature (°C) | 200 |
| Weight Loss after pre-calcination (wt.%) | 4.36% |
| Activation temperature (°C) | 482 |
| Isomerization Selectivity at 96% Conversion | 83.6% |
| Temperature (°F) | 532 |
| $C_4$- Cracking (%) | 1.9% |

**EXAMPLE 13**

Palladium ion-exchange was carried out on the ammonium-exchanged sample from Example 6 per the teachings of Example 8. The palladium-exchanged sample was tested for the selective hydroconversion of n-hexadecane under the conditions described in Example 8. The results are presented in Table 6. The isomerization selectivity at 96% conversion for this sample is better than those produced by prior art and conventional methods for the conversion of the n-$C_{16}$ feed to isomerized products, with lower gas make due to n-$C_{16}$ cracking.

TABLE 6

| | |
|---|---|
| Pre-calcination Temperature (°C) | 250 |
| Weight Loss after pre-calcination (wt.%) | 4.73 |
| Activation temperature (°C) | 482 |
| Isomerization Selectivity at 96% Conversion | 83.6% |
| Temperature (°F) | 532 |
| $C_{4-}$ Cracking (%) | 1.9 |

**EXAMPLE 14 (Comparative)**

Palladium ion-exchange was carried out on the ammonium-exchanged sample from Example 7 per the teachings of Example 8. The palladium-exchanged sample was tested for the selective hydroconversion of n-hexadecane under the conditions described in Example 8. The results are shown in Table 7. The isomerization selectivity at 96% conversion for this sample is inferior to those described by the present invention and presented in Examples 8 to 13, as indicated in Table 8.

TABLE 7

| | |
|---|---|
| Pre-calcination Temperature (°C) | 595 |
| Weight Loss after pre-calcination (wt.%) | 12.23 |
| Micropore Vol. (cc/g) | 0.0414 |
| Ext. area ($m^2$/g) | 235.9 |
| BET area ($m^2$/g) | 330.8 |
| Activation temperature (°C) | 482 |
| Isomerization Selectivity at 96% Conversion | 81.2% |
| Temperature (°F) | 533 |
| $C_{4-}$ Cracking (%) | 2.30 |

As shown in Table 8 below, by maintaining the pre-calcination temperature between 200 and 400°C, with a weight loss of below 10 wt.%, enhanced isomerization selectivity was achieved as compared to material that was subjected to conventional calcination temperatures. In addition, the samples were subjected to pre-calcination temperature between 200 and 400°C, the products exhibited a significantly lower micropore volume after ammonium exchange as compared to the sample prepared using a conventional, higher pre-calcination temperature (Comparative Example 14). The lower micropore volume is indicative of the presence of decomposition residue in the pores of the molecular sieve resulting in the preferred acid site density and location of these sites.

TABLE 8

| Example | Isomerization Selectivity at 96% Conversion | Pre-calcination Temperature (°C) | Micropore Vol. (cc/g) | Weight Loss after pre-calcination (wt.%) |
|---|---|---|---|---|
| 8 | 83% | 300 | 0.0130 | 5.02 |
| 9 | 82.3% | 320 | 0.0133 | 5.17 |
| 10 | 84.4% | 350 | 0.0135 | 5.72 |
| 11 | 83.4% | 400 | 0.0141 | 8.23 |
| 12 | 83.6% | 200 | ----- | 4.36 |
| 13 | 83.6% | 250 | ----- | 4.73 |
| 14 (conventional) | 81.2% | 595* | 0.0414 | 12.23 |

* Temperature results in full calcination of the sample

**EVALUATION BY ZEOLITE ACIDITY MEASUREMENTS**

**EXAMPLE 15**

A 50 g of as-synthesized SSZ-32x was pre-calcined following the procedure described in Example 4. The pre-calcined sample was exchanged into the ammonium form following the teachings of Example 1. Next, the ammonium-exchanged material was post-calcined in a muffle furnace under an atmosphere of dry air at a heating rate of 1°C/min. to 120°C and held for 180 min followed by a second ramp of 1°C/min. to 400°C and held at this temperature for 180 min. The zeolite was then cooled to ambient temperature before acidity measurement by FTIR and micropore analysis. Prior to FTIR measurement, the sample was heated for 1 hour at 400-450 °C under vacuum <1x$10^{-5}$ Torr. After cooling down to 150°C, dosage of deuterated benzene was added until H-D exchange equilibrium was reached. Then FTIR was recorded in OD stretching region. Acidity was determined by the amount of bridged hydroxyl groups exchanged with deuterated benzene at 150°C. The sample had a micropore volume of 0.0132 cc/g, external surface area of 217.66 $m^2$/g and BET surface area of 251.76 $m^2$/g.

**EXAMPLE 16**

A 50 g of as-synthesized SSZ-32x zeolite sample was exchanged into the ammonium form following the teachings of Example 1. Sample was washed with copious amount of deionized water to a conductivity less than 50 $\mu$S/cm and finally dried in an oven at 120°C for three hours. Then the dried sample was post-calcined in a muffle furnace under an atmosphere of dry air at a heating rate of 1°C/min. to 120°C and held for 180 min followed by a second ramp of 1°C/min. to 482°C and held at this temperature for 180 min. The zeolite was then cooled to ambient temperature before acidity measurement by FTIR. Then, acidity was determined by the amount of bridged hydroxyl groups exchanged with deuterated benzene at 150°C following the teachings of Example 15.

**EXAMPLE 17**

A 50 g of as-synthesized SSZ-32x zeolite sample was pre-calcined and exchanged into the ammonium form following the procedure described in Example 3. Then the sample was post-calcined in a muffle furnace under an atmosphere of dry air at a heating rate of 1°C/min. to 120°C and held for 180 min followed by a second ramp of 1°C/min. to 482°C and held at this temperature for 180 min. The zeolite was then cooled to ambient temperature before acidity measurement by FTIR. Acidity was determined by the amount of bridged hydroxyl groups exchanged with deuterated benzene at 150°C following the teachings of Example 15.

**EXAMPLE 18**

A 50 g of as-synthesized SSZ-32x sample was pre-calcined and exchanged into the ammonium form following the procedure described in Example 4. Then the sample was post-calcined following the procedure described in Example 17. Acidity was determined by the amount of bridged hydroxyl groups exchanged with deuterated benzene at 150°C. The sample had a micropore volume of 0.0144 cc/g, external surface area of 231.78 $m^2$/g and BET surface area of 268.21 $m^2$/g.

## EXAMPLE 19

A 40 g of as-synthesized SSZ-32x sample was calcined in a muffle furnace under an atmosphere of dry air at a heating rate of 1°C/min. to 120°C and held for 120 min followed by a second ramp of 1°C/min. to 450°C and held at this temperature for 180 min. Finally, the sample was cooled down to 150°C. The calcined sample was then exchanged into the ammonium form following the procedure described in Example 1. Sample was washed with copious amount of deionized water to a conductivity less than 50 μS/cm and finally dried in an oven at 120°C for three hours. Then the sample was post-calcined following the procedure described in Example 17. Acidity was determined by the amount of bridged hydroxyl groups exchanged with deuterated benzene at 150°C following the teachings of Example 15.

## COMPARATIVE EXAMPLE 20

Example 20 was prepared using standard SSZ-32 zeolite, which was calcined at 600 °C prior to ammonium ion-exchange and dried only at 120 °C after ammonium ion-exchange. Acidity was determined by the amount of bridged hydroxyl groups exchanged with deuterated benzene at 150°C following the teachings of Example 15.

## COMPARATIVE EXAMPLE 21

As-synthesized SSZ-32x sample was calcined at 595 °C prior to ammonium ion-exchange and dried only at 120 °C after ammonium ion-exchange following the procedure described in Example 7. Acidity was determined by the amount of bridged hydroxyl groups exchanged with deuterated benzene at 150°C following the teachings of Example 15.

## CATALYST PREPARATION AND EVALUATION

## EXAMPLE 22

A catalyst was prepared using zeolite from Example 15 according to the method disclosed in US 7,468,126 B2 to Zones et al., granted on Dec 23, 2008. The dried and calcined extrudate was impregnated with a solution containing platinum. The overall platinum loading was 0.325 wt.%.

### Real feed performance test conditions

A feed "light neutral" (LN) was used to evaluate the invented catalysts. Properties of the feed are listed in the following Table 9.

TABLE 9

| | |
|---|---|
| API Gravity | 34 |
| N, ppm | <0.3 |
| S, ppm | 6 |
| VI | 120 |
| Vis@100°C (cSt) | 3.92 |
| Vis@70°C (cSt) | 7.31 |
| Wax (wt%) | 12.9 |
| Dewaxed oil properties | |
| DWO VI | 101 |
| DWO Vis@100°C (cSt) | 4.08 |
| DWO Vis@40°C (cSt) | 20.1 |
| SIMDIST TBP (wt%) (°F) | |
| TBP @0.5 | 536 |
| TBP @5 | 639 |
| TBP @10 | 674 |
| TBP @30 | 735 |
| TBP @50 | 769 |
| TBP @70 | 801 |
| TBP @90 | 849 |

(continued)

| | |
|---|---|
| SIMDIST TBP (wt%) (°F) | |
| TBP @95 | 871 |
| TBP @99.5 | 910 |

The reaction was performed in a micro unit equipped with two fix bed reactors. The run was operated under 2100 psig total pressure. Prior to the introduction of feed, the catalysts were activated by a standard reduction procedure. The LN feed was passed through the hydroisomerization reactor at a LHSV of 2 and then was hydrofinished in the 2nd reactor, which was loaded with a Pd/Pt catalyst to further improve the lube product quality. The hydrogen to oil ratio was about 3000 scfb. The lube product was separated from fuels through the distillation section. Pour point, cloud point, viscosity, viscosity index and simdist were collected on the products.
The real feed test results are presented in Table 11.

**EXAMPLE 23**

A catalyst was prepared using zeolite from Example 16 by following the procedure described in Example 22.

**EXAMPLE 24**

A catalyst was prepared using zeolite from Example 17 by following the procedure described in Example 22.

**EXAMPLE 25**

A catalyst was prepared using zeolite from Example 18 by following the procedure described in Example 22.

**EXAMPLE 26**

A catalyst was prepared using zeolite from Example 19 by following the procedure described in Example 22.

**COMPARATIVE EXAMPLE 27**

Example 27 was a dewaxing catalyst containing standard SSZ-32 zeolite from Example 20. A catalyst was prepared by following the procedure described in Example 22.

**COMPARATIVE EXAMPLE 28**

Example 28 was a dewaxing catalyst containing standard SSZ-32X zeolite from Example 21. A catalyst was prepared by following the procedure described in Example 22.

**EXAMPLE 29**

Table 10 shows the FTIR stretching results of the zeolite component (after exchange with deuterated benzene) in Examples 15-21 and Table 11 shows the lube dewaxing data for Examples 22-28.
According to FTIR results, Table 10 shows about 32% acid site density present in the zeolite component of Example 19 relative to Comparative Example 20. The acid site density was decreased from 13.4% to 11.1% when the pre-calcination temperature was decreased from 400 °C (Example 18) to 350 °C (Example 17). Examples 15 and 16, which produced the highest lube yield and with the best activity, (See Table 11) have the lowest acid site density: 3.1% and 5.9% respectively. This suggests the catalysts performance is inversely proportional to the amount of active acid sites in the zeolite component.
Compared to Comparative Example 27, all catalysts in this invention showed significantly improved lube yield, activity and VI as shown in Table 11. Examples 24 and 26 gained -29 °F in activity when the yield was increased by 3.9 wt% and 2.1 wt% respectively. The VI was increased by 1.5 and 1.6 respectively. For Examples 22 and 23, both lube yield and activity were further improved to 4-4.4 wt% and 34 °F respectively.

TABLE 10

| Catalyst | Comparative Example 20 (SSZ-32) | Comparative Example 21 (SSZ-32x) | Example 15 | Example 16 | Example 17 | Example 18 | Example 19 |
|---|---|---|---|---|---|---|---|
| Pre-calcination temperature (°C) | 600 | 595 | 400 | 120 | 350 | 400 | 450 |
| Post-calcination temperature (°C) | 120 | 120 | 400 | 482 | 482 | 482 | 482 |
| Acid sites in zeolite determined by H-D exchange (relative to zeolite SSZ-32)* (%) | 100.0 | 55.7 | 3.1 | 5.9 | 11.1 | 13.4 | 31.9 |
| Micropore Vol. (cc/g) | 0.0601 | 0.029 | 0.0132 | ----- | ----- | 0.0144 | ----- |
| *For FTIR measurement, the sample was heated for 1 hour at 400-450 °C under vacuum <1x$10^{-5}$ Torr | | | | | | | |

TABLE 11

| Catalyst | Comparative Example 27 (SSZ-32) | Comparative Example 28 (SSZ-32x) | Example 22 | Example 23 | Example 24 | Example 25 | Example 26 |
|---|---|---|---|---|---|---|---|
| CAT at pour point (-15 °C) | Base | -39.3 | -34.0 | -34.0 | -29.3 | - | -29.1 |
| Lube yield (wt. %) | Base | +1.1 | +4.0 | +4.4 | +3.9 | - | +2.1 |
| Gas (wt. %) | Base | -1.2 | -1.7 | -1.6 | -1.6 | - | -1.3 |
| Viscosity Index | Base | +0 | +3.2 | +1.5 | +1.5 | - | +1.6 |

## Claims

**1.** A process for converting hydrocarbons comprising contacting a hydrocarbonaceous feed under hydrocarbon converting conditions with a catalyst comprising a molecular sieve, the molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure

directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**2.** The process of Claim 1, wherein the molecular sieve has a mole ratio of 20 to 50 of silicon oxide to aluminum oxide.

**3.** The process of Claim 1, wherein the molecular sieve has a total micropore volume of between 0.008 and 0.018 cc/g.

**4.** The process of Claim 1, wherein the molecular sieve has an external surface area of between 200 and 250 $m^2/g$; and a BET surface area of between 240 and 280 $m^2/g$.

**5.** The process of Claim 1, wherein the molecular sieve has a H-D exchangeable acid site density of 0.5 to 30% relative to molecular sieve SSZ-32.

**6.** The process of Claim 1, wherein the molecular sieve has a H-D exchangeable acid site density of 2 to 25% relative to molecular sieve SSZ-32.

**7.** The process of Claim 1, wherein the process is a process selected from the group consisting of hydrocracking, dewaxing, catalytic cracking, aromatics formation, isomerization, alkylation and transalkylation, conversion of paraffins to aromatics, isomerization of olefins, xylene isomerization, oligomerization, condensation of alcohols, methane upgrading and polymerization of 1-olefins.

**8.** The process of Claim 7, wherein the process is a dewaxing process comprising contacting the catalyst with a hydrocarbon feedstock under dewaxing conditions.

**9.** The process of Claim 1, wherein the process is a process for producing a $C_{20+}$ lube oil from a $C_{20+}$ olefin feed comprising isomerizing the olefin feed under isomerization conditions over the catalyst.

**10.** A process for the production of light olefins from a feedstock comprising an oxygenate or mixture of oxygenates, the process comprising reacting the feedstock at effective conditions over a catalyst comprising a molecular sieve, the molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**11.** A process for producing methylamine or dimethylamine comprising reacting methanol, dimethyl ether or a mixture thereof and ammonia in the gaseous phase in the presence of a catalyst comprising a molecular sieve, the molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;

(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**12.** In a process for separating gases using a membrane containing a molecular sieve, the improvement comprising using as the molecular sieve a molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**13.** A process for treating a cold-start engine exhaust gas stream containing hydrocarbons and other pollutants consisting of flowing the engine exhaust gas stream over a molecular sieve bed which preferentially adsorbs the hydrocarbons over water to provide a first exhaust stream, and flowing the first exhaust gas stream over a catalyst to convert any residual hydrocarbons and other pollutants contained in the first exhaust gas stream to innocuous products and provide a treated exhaust stream and discharging the treated exhaust stream into the atmosphere, the molecular sieve bed comprising a molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;
(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**14.** A process for the reduction of oxides of nitrogen contained in a gas stream wherein the process comprises contacting the gas stream with a molecular sieve, the molecular sieve having a MTT-type framework, a mole ratio of 20 to 70 of silicon oxide to aluminum oxide, a total micropore volume of between 0.005 and 0.02 cc/g; and a H-D exchangeable acid site density of up to 50% relative to SSZ-32, wherein the molecular sieve is obtainable by:

(a) providing as-made, structure directing agent-containing molecular sieve SSZ-32x having a silicon-to-alumina ratio of 20 to 70;
(b) subjecting the molecular sieve to a pre-calcination step at a temperature below the full decomposition temperature of the structure directing agent, for a time sufficient to convert at least a portion of the structure directing agent to a decomposition residue;

(c) ion-exchanging the pre-calcined molecular sieve to remove extra-framework cations; and
(d) subjecting the molecular sieve to a post-calcination step at a temperature below the full decomposition temperature of the structure directing agent, for a time sufficient to convert at least a portion of the structure directing agent to a decomposition residue;
wherein the post-calcined molecular sieve has a cumulative weight loss (CWL) of $0 < CWL \leq 10$ wt.% and a total micropore volume of between 0.005 and 0.02 cc/g.

**Patentansprüche**

1. Verfahren für die Umwandlung von Kohlenwasserstoffen, umfassend Zusammenbringen einer kohlenwasserstoffhaltigen Beschickung unter Kohlenwasserstoffumwandlungsbedingungen mit einem Katalysator, umfassend ein Molekularsieb, wobei das Molekularsieb ein Gerüst vom MTT-Typ, ein Molverhältnis von 20 bis 70 von Siliziumoxid zu Aluminiumoxid, und ein Gesamtmikroporenvolumen zwischen 0,005 und 0,02 $cm^3/g$ hat; und eine H-D austauschbare Säurestellendichte von bis zu 50% relativ zu SSZ-32, worin das Molekularsieb erhalten werden kann durch:

   (a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
   (b) Aussetzen des Molekularsiebs einem Vorkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
   (c) Ionenaustausch des vorkalzinierten Molekularsiebs, um außerhalb des Gerüsts befindliche Kationen zu entfernen;
   (d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
   worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

2. Verfahren gemäß Anspruch 1, worin das Molekularsieb ein Molverhältnis von 20 bis 50 von Siliziumoxid zu Aluminiumoxid hat.

3. Verfahren gemäß Anspruch 1, worin das Molekularsieb ein Gesamtmikroporenvolumen zwischen 0,008 und 0,018 $cm^3/g$ hat.

4. Verfahren gemäß Anspruch 1, worin das Molekularsieb eine äußere Oberfläche zwischen 200 und 250 $m^2/g$ hat; und eine BET-Oberfläche zwischen 240 und 280 $m^2/g$.

5. Verfahren gemäß Anspruch 1, worin das Molekularsieb eine H-D austauschbare Säurestellendichte von 0,5 bis 30% relativ zum Molekularsieb SSZ-32 hat.

6. Verfahren gemäß Anspruch 1, worin das Molekularsieb eine H-D austauschbare Säurestellendichte von 2 bis 25% relativ zum Molekularsieb SSZ-32 hat.

7. Verfahren gemäß Anspruch 1, worin das Verfahren ein Verfahren ist, ausgewählt aus der Gruppe Hydrocracken, Entparaffinierung, katalytisches Cracken, Aromatenbildung, Isomerisierung, Alkylierung und Transalkylierung, Umwandlung von Paraffinen zu Aromaten, Olefinisomerisierung, Xylolisomerisierung, Oligomerisierung, Alkoholkondensation, Methanveredlung und 1-Olefinpolymerisierung.

8. Verfahren gemäß Anspruch 7, worin das Verfahren ein Entparaffinierungsverfahren ist, umfassend Zusammenbringen des Katalysators mit einer Kohlenwasserstoffbeschickung unter Entparaffinierungsbedingungen.

9. Verfahren gemäß Anspruch 1, worin das Verfahren ein Verfahren für die Herstellung eines $C_{20+}$-Schmieröls aus einer $C_{20+}$-Olefinbeschickung ist, umfassend Isomerisieren der Olefinbeschickung unter Isomerisierungsbedingungen über dem Katalysator.

10. Verfahren für die Herstellung leichter Olefine aus einem ein Oxygenat oder eine Mischung von Oxygenaten umfassenden Ausgangsmaterial, wobei das Verfahren die Reaktion des Ausgangsmaterials bei effektiven Bedingungen über einem ein Molekularsieb umfassenden Katalysator umfasst, wobei das Molkularsieb ein Gerüst vom MTT-Typ, ein Molverhältnis von 20 bis 70 von Siliziumoxid zu Aluminiumoxid, und ein Gesamtmikroporenvolumen zwischen 0,005 und 0,02 $cm^3/g$ hat; und eine H-D austauschbare Säurestellendichte von bis zu 50% relativ zu SSZ-32, worin das Molekularsieb erhalten werden kann durch:

(a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
(b) Aussetzen des Molekularsiebs einem Vorkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
(c) Ionenaustausch des vorkalzinierten Molekularsiebs, um außerhalb des Gerüsts befindliche Kationen zu entfernen;
(d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

**11.** Verfahren für die Herstellung von Methylamin oder Dimethylamin, umfassend die Reaktion von Methanol, Dimethylether oder einer Mischung davon und Ammoniak in der Gasphase, in der Anwesenheit eines ein Molekularsieb umfassenden Katalysators, wobei das Molekularsieb ein Gerüst vom MTT-Typ, ein Molverhältnis von 20 bis 70 von Siliziumoxid zu Aluminiumoxid, ein Gesamtmikroporenvolumen zwischen 0,005 und 0,02 $cm^3/g$ hat; und eine H-D austauschbare Säurestellendichte von bis zu 50% relativ zu SSZ-32, worin das Molekularsieb erhalten werden kann durch:

(a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
(b) Aussetzen des Molekularsiebs einem Vorkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
(c) Ionenaustausch des vorkalzinierten Molekularsiebs, um außerhalb des Gerüsts befindliche Kationen zu entfernen;
(d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

**12.** Verwendung einer ein Molekularsieb enthaltenden Membran in einem Verfahren für die Auftrennung von Gasen, die Verbesserung umfassend die Verwendung, als das Molekularsieb, eines Molekularsiebs mit einem Gerüst vom MTT-Typ, einem Molverhältnis von 20 bis 70 von Siliziumoxid zu Aluminiumoxid, einem Gesamtmikroporenvolumen zwischen 0,005 und 0,02 $cm^3/g$ hat; und einer H-D austauschbaren Säurestellendichte von bis zu 50% relativ zu SSZ-32, worin das Molekularsieb erhalten werden kann durch:

(a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
(b) Aussetzen des Molekularsiebs einem Vorkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
(c) Ionenaustausch des vorkalzinierten Molekularsiebs, um außerhalb des Gerüsts befindliche Kationen zu entfernen;
(d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

**13.** Verfahren für die Behandlung eines Kohlenwasserstoffe und andere Schadstoffe enthaltenden Abgasstroms eines Kaltstartmotors, bestehend aus dem Strömenlassen des Motorabgasstroms über ein Molekularsiebbett, das bevorzugt die Kohlenwasserstoffe über Wasser adsorbiert, um einen ersten Abgasstrom bereitzustellen, und dem Strömenlassen des ersten Abgasstroms über einen Katalysator, um etwaige Restkohlenwasserstoffe und andere Schadstoffe, welche im ersten Abgasstrom enthalten sind, in unschädliche Produkte umzuwandeln und einen behandelten Abgasstrom bereitzustellen, und dem Ausstoßen des behandelten Abgasstroms in die Atmosphäre, wobei das Molekularsiebbett ein Molekularsieb umfasst, mit einem Gerüst vom MTT-Typ, einem Molverhältnis von 20 bis 70 von Siliziumoxid zu Aluminiumoxid, einem Gesamtmikroporenvolumen zwischen 0,005 und 0,02 $cm^3/g$; und einer H-D austauschbaren Säurestellendichte von bis zu 50% relativ zu SSZ-32, worin das Molekularsieb erhalten werden kann durch:

(a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
(b) Aussetzen des Molekularsiebs einem Vorkalzinierungsschritt bei einer Temperatur unterhalb der vollständigen Zersetzungstemperatur des Struktursteuerungsmittels, für eine Zeit ausreichend, um mindestens einen Teil des Struktursteuerungsmittels in einen Zersetzungsrückstand umzuwandeln;
(c) Ionenaustausch des vorkalzinierten Molekularsiebs, um außerhalb des Gerüsts befindliche Kationen zu entfernen;
(d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

**14.** Verfahren zur Reduktion von in einem Gasstrom enthaltenen Stickstoffoxiden, worin das Verfahren Zusammenbringen des Gasstroms mit einem Molekularsieb umfasst, wobei das Molekularsieb ein Gerüst vom MTT-Typ, ein Molverhältnis von 20 bis 70 von Siliziumoxid zu Aluminiumoxid, und ein Gesamtmikroporenvolumen zwischen 0,005 und 0,02 $cm^3/g$ hat; und eine H-D austauschbare Säurestellendichte von bis zu 50% relativ zu SSZ-32, worin das Molekularsieb erhalten werden kann durch:

(a) Bereitstellen, wie hergestellt, eines ein Struktursteuerungsmittel enthaltenden Molekularsiebs SSZ-32x mit einem Silizium-zu-Aluminiumoxid-Verhältnis von 20 bis 70;
(b) Aussetzen des Molekularsiebs einem Vorkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
(c) Ionenaustausch des vorkalzinierten Molekularsiebs, um außerhalb des Gerüsts befindliche Kationen zu entfernen;
(d) Aussetzen des Molekularsiebs einem Nachkalzinierungsschritt bei einer Temperatur unterhalb der vollständigen Zersetzungstemperatur des Struktursteuerungsmittels, für eine Zeit, ausreichend, um mindestens einen Teil des Struktursteuerungsmittels in einen Zersetzungsrückstand umzuwandeln;
worin das nachkalzinierte Molekularsieb einen kumulativen Gewichtsverlust (CWL) von $0 < CWL \leq 10$ Gew.-% und ein Gesamtmikroporenvolumen zwischen 0,005 und 0,02 $cm^3/g$ hat.

## Revendications

**1.** Un procédé pour la conversion d'hydrocarbures comprenant la mise en contact d'une alimentation hydrocarbonée dans des conditions de conversion d'hydrocarbures avec un catalyseur comprenant un tamis moléculaire, le tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et

(d) soumettre le tamis moléculaire à une étape de post-calcination à une température inférieure à la température de décomposition complète de l'agent directeur de structure, pendant un temps suffisant pour convertir au moins une partie de l'agent directeur structure en un résidu de décomposition;
dans lequel le tamis moléculaire post-calciné a une perte de poids cumulée (CWL) de $0 < CWL \leq 10\%$ en poids et un volume total de micropores entre 0,005 et 0,02 $cm^3/g$.

**2.** Le procédé selon la revendication 1, dans lequel le tamis moléculaire a un rapport molaire de 20 à 50 de l'oxyde de silicium par rapport par rapport à l'oxyde d'aluminium.

**3.** Le procédé selon la revendication 1, dans lequel le tamis moléculaire a un volume total de micropores compris entre 0,008 et 0,018 cc/g.

**4.** Le procédé selon la revendication 1, dans lequel le tamis moléculaire a une surface extérieure comprise entre 200 et 250 $m^2/g$; et une surface BET comprise entre 240 et 280 $m^2/g$.

**5.** Le procédé selon la revendication 1, dans lequel le tamis moléculaire a une densité de sites acides échangeables H-D de 0,5 à 50% par rapport au tamis moléculaire SSZ-32.

**6.** Le procédé selon la revendication 1, dans lequel le tamis moléculaire a une densité de sites acides échangeables H-D de 2 à 25% par rapport au tamis moléculaire SSZ-32.

**7.** Le procédé selon la revendication 1, dans lequel le procédé est un procédé choisi parmi le groupe constitué en l'hydrocraquage, le déparaffinage, le craquage catalytique, la formation d'aromatiques, l'isomérisation, l'alkylation et la transalkylation, la conversion de paraffines en aromatiques, l'isomérisation d'oléfines, l'isomérisation du xylène, l'oligomérisation, la condensation des alcools, la valorisation du méthane et la polymérisation des 1-oléfines.

**8.** Le procédé selon la revendication 7, dans lequel le procédé est un procédé de déparaffinage comprenant la mise en contact du catalyseur avec une charge d'alimentation d'hydrocarbures dans des conditions de déparaffinage.

**9.** Le procédé selon la revendication 1, dans lequel le procédé est un procédé pour la production d'une huile lubrifiante en $C_{20+}$ à partir d'une alimentation d'oléfines en $C_{20+}$ comprenant l'isomérisation de l'alimentation d'oléfines dans des conditions d'isomérisation sur le catalyseur.

**10.** Un procédé pour la production d'oléfines légères à partir d'une charge d'alimentation comprenant un composé oxygéné ou un mélange de composés oxygénés, le procédé comprenant la réaction de la charge d'alimentation dans des conditions efficaces sur un catalyseur comprenant un tamis moléculaire, le tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et
(d) soumettre le tamis moléculaire à une étape de post-calcination à une température inférieure à la température de décomposition complète de l'agent directeur de structure, pendant un temps suffisant pour convertir au moins une partie de l'agent directeur de structure en un résidu de décomposition;
dans lequel le tamis moléculaire post-calciné a une perte de poids cumulée (CWL) de $0 < CWL \leq 10\%$ en poids et un volume total de micropores compris entre 0,005 et 0,02 $cm^3/g$.

**11.** Un procédé pour la production de méthylamine ou de diméthylamine comprenant la réaction de méthanol, d'éther diméthylique ou leurs mélange et d'ammoniac en phase gazeuse en présence d'un catalyseur comprenant un tamis moléculaire, le tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et
(d) soumettre le tamis moléculaire à une étape de post-calcination à une température inférieure à la température de décomposition complète de l'agent directeur de structure, pendent un temps suffisant pour convertir au moins une partie de l'agent directeur structure en un résidu de décomposition;
dans lequel le tamis moléculaire post-calciné a une perte de poids cumulée (CWL) de $0 < CWL \leq 10\%$ en poids et un volume total de micropores compris entre 0,005 et 0,02 $cm^3/g$.

**12.** Dans un procédé pour séparer des gaz en utilisant une membrane contenant un tamis moléculaire, l'amélioration comprenant utiliser comme le tamis moléculaire un tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et
(d) soumettre le tamis moléculaire à une étape de post-calcination à une température inférieure à la température de décomposition complète de l'agent directeur de structure, pendent un temps suffisant pour convertir au moins une partie de l'agent directeur structure en un résidu de décomposition;
dans lequel le tamis moléculaire post-calciné a une perte de poids cumulée (CWL) de $0 < CWL \leq 10\%$ en poids et un volume total de micropores compris entre 0,005 et 0,02 $cm^3/g$.

**13.** Un procédé pour le traitement d'un flux de gaz d'échappement d'un moteur de démarrage à froid contenant des hydrocarbures et d'autres polluant consistant à faire circuler le flux de gaz d'échappement de moteur sur un lit de tamis moléculaire qui adsorbe préférentiellement les hydrocarbures sur l'eau pour fournir un premier flux d'échappement, et faire circuler le premier flux de gaz d'échappement sur un catalyseur pour convertir tous les hydrocarbures résiduels et autres polluants contenus dans le premier flux de gaz d'échappement en produits inoffensifs et fournir un flux d'échappement traité et décharger le flux d'échappement dans l'atmosphère, le lit de tamis moléculaire comprenant un tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et
(d) soumettre le tamis moléculaire à une étape de post-calcination à une température inférieure à la température de décomposition complète de l'agent directeur de structure, pendent un temps suffisant pour convertir au moins une partie de l'agent directeur structure en un résidu de décomposition;
dans lequel le tamis moléculaire post-calciné a une perte de poids cumulée (CWL) de $0 < CWL \leq 10\%$ en poids et un volume total de micropores compris entre 0,005 et 0,02 $cm^3/g$.

**14.** Un procédé pour la réduction des oxydes d'azote contenus dans un flux de gaz, dans lequel le procédé comprend la mise en contact du flux de gaz avec un tamis moléculaire, le tamis moléculaire ayant un cadre de type MTT, un rapport molaire de 20 à 70 de l'oxyde de silicium par rapport à l'oxyde d'aluminium, un volume total de micropores compris entre 0,005 à 0,02 $cm^3/g$; et une densité de sites acides échangeables H-D allant jusqu'au 50% par rapport à SSZ-32, dans lequel le tamis moléculaire peut être obtenu par:

(a) fournir, comme fabriqué, un tamis moléculaire SSZ-32x contenant un agent directeur de structure ayant un rapport de silicium par rapport à l'oxyde d'aluminium de 20 à 70;
(b) soumettre le tamis moléculaire à une étape de pré-calcination à une température inférieure à la température de décomposition complète de l'agent directeur de structure, pendant un temps suffisant pour convertir au moins une partie de l'agent directeur structure en un résidu de décomposition;
(c) échanger les ions du tamis moléculaire pré-calciné pour éliminer des cations extra-cadre; et
(d) soumettre le tamis moléculaire à une étape de post-calcination à une température inférieure à la température de décomposition complète de l'agent directeur de structure, pendent un temps suffisant pour convertir au moins une partie de l'agent directeur structure en un résidu de décomposition;
dans lequel le tamis moléculaire post-calciné a une perte de poids cumulée (CWL) de $0 < CWL \leq 10\%$ en poids et un volume total de micropores compris entre 0,005 et 0,02 $cm^3/g$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7390763 B
- US 7569507 B
- US 8545805 B
- WO 2005042144 A
- WO 2007146622 A
- US 6790433 B, C.Y. Chen and Stacey Zones
- US 5252527 A, Zones
- US 5053373 A
- US 5397454 A
- US 5707601 A
- US 5785947 A
- US 6099820 A
- US 7157075 B
- US 7468126 B
- US 7682600 B
- US 7824658 B
- US 4910006 A
- US 5316753 A
- US 4094821 A
- US 4347121 A
- US 4810357 A
- US 3904513 A
- US 4157294 A
- US 3852207 A
- US 4921594 A
- US 5149421 A
- US 4181598 A
- US 4734537 A
- US 4939311 A
- US 4962261 A
- US 5095161 A
- US 5105044 A
- US 5105046 A
- US 5238898 A
- US 5321185 A
- US 5336825 A
- US 5082990 A
- US 3960978 A
- US 4376722 A
- US 4377497 A
- US 4446243 A
- US 4526942 A
- US 3709853 A
- US 4086408 A
- US 3894107 A
- US 4410501 A
- US 4677242 A
- US 4861938 A
- US 6459000 B
- US 6548722 B
- US 6630606 B
- US 4737592 A
- EP 234088 A
- US 4883915 A
- US 5942613 A
- US 6508860 B
- US 2942932 A
- US 3699683 A
- US 5078979 A
- US 3920583 A
- US 3767453 A
- US 3785998 A
- US 4791091 A
- US 4528279 A
- US 4760044 A
- US 4868148 A
- US 4868149 A
- US 4297328 A
- US 7468126 B2, Zones

### Non-patent literature cited in the description

- Atlas of Zeolite Framework Types. Elsevier, 2007
- *Chem. Eng. News,* 1985, vol. 63 (5), 26-27
- **E.J.M HENSEN ; D. G. PODUVAL ; D.A.J MICHEL LIGTHART ; J.A. ROB VAN VEEN ; M. S. RIGUTTO.** *J.Phys. Chem.,* 2010, vol. C.114, 8363-8374
- **D.R.C. HUYBRECHTS et al.** *Nature,* 1990, vol. 345, 240-242
- **T. TATSUMI et al.** *J. Chem. Soc. Chem. Commun.,* 1990, 476-477